# EUROPÄISCHE PATENTSCHRIFT

(11) **EP 0 446 485 B1**
(45) Veröffentlichungstag und Bekanntmachung des Hinweises auf die Patenterteilung: **06.09.1995**
(21) Anmeldenummer: 90125411.0
(22) Anmeldetag: 24.12.1990
(51) Int. Cl.: C07K 5/02, C07K 5/04, A61K 38/55, C07D 339/08, A61K 31/385

(54) **Peptid-Analoga mit renininhibierender Aktivität**
Peptide analogues with renininhibiting activity
Analogues peptidiques ayant une activité inhibante de rénine

(30) Priorität: 16.02.1990 DE 4004898
(43) Veröffentlichungstag der Anmeldung: 18.09.1991
(73) Patentinhaber: MERCK PATENT GmbH, D-64271 Darmstadt (DE)
(72) Erfinder: Juraszyk, Horst, Dr., W-6104 Seeheim (DE); Raddatz, Peter, Dr., W-6104 Seeheim (DE); Sombroek, Johannes, Dr., W-6100 Darmstadt (DE); Schmitges, Claus J., Dr., W-6114 Gross-Umstadt (DE); Minck, Klaus-Otto, Dr., W-6105 Ober-Ramstadt (DE)

(56) Entgegenhaltungen:
- EP-A- 0 273 893
- JOURNAL OF MEDICINAL CHEMISTRY Bnd. 31, 1988, WASHINGTON, USA Seite 625 - 629;MAIBAUM ET AL: 'Inhibition of porcine pepsin by two substrate analoguescontaining statine: The effect of histidine at the P2 subsite on the inhibitionof aspartic proteinases'
- CHEMICAL ABSTRACTS, Bnd. 111, 1989, Columbus, Ohio, US; Zusammenfassung 233677,WILLIAMS ET AL: 'Di- or tripeptiderenin inhibitors containing conformationally restricted4-amino-5-cyclohexyl-3-hydroxypentanoic acid (ACHPA) analogs' Seite 866; Spalte 1.

## Beschreibung

Die Erfindung betrifft neue Peptid-Analoga der Formel I

R¹-Z―NR²-CHR³-CR⁴-(CH₂)ₒ-(CR⁵)ₜ-CE-C_{w}H_{2w}-R⁶

worin
- R¹: H, R⁷-CₘH₂ₘ-O-CO-, R⁷-CₘH₂ₘ-CO-, R⁸R⁹N-CₘH₂ₘ-CO-, oder R¹¹-CₘH₂ₘ-(T)ₛ-(V)_{y}-CₙH₂ₙ-L(R⁷-CₚH₂ₚ)-CᵣH₂ᵣ-CO-,
- Z: 0 bis 2 peptidartig miteinander verbundene Aminosäurereste ausgewählt aus der Gruppe bestehend aus βAla, Asn, Asp, Cal, S-A-Cys, Gln, His, Ile, Isoser, Leu, Mal, Met, Met(O₂), Nle, Nva, Phe, Ser, Thr und Val, wobei an Stelle eines dieser Reste auch Pla stehen kann,
- R²: H,
- R³: Cyclohexylmethyl,
- R⁴: (H, R¹²)
- R⁵: (H, H) oder (H, OH),
- R⁶: H, A oder COOR⁷,
- R⁷ und R¹¹: jeweils H, A, Ar oder Het,
- R⁸R⁹N: eine unsubstituierte oder eine durch NH₂ oder NH-CO-O-A substituierte Piperidino- oder Morpholinogruppe,
- R¹¹: auch R⁸OOC-,
- R¹²: OH,
- R² und R¹²: zusammen auch -C(A)₂-O-,
- E: -S(O)_{b}-(CH₂)₃-S(O)_{b}-,
- L: CH oder N,
- T: NH,
- V: S oder SO₂,
- a: 2 oder 3,
- b: 0 oder 2,
- s, t und y: jeweils 0 oder 1,
- m, n, o, p und w: jeweils 0, 1, 2, 3 oder 4,
- Ar: unsubstituiertes oder einfach durch Hal, substituiertes Phenyl,
- Het: einen gesättigten oder ungesättigten 5- oder 6-gliedrigen heterocyclischen Rest mit 1-4 N-Atomen, der einfach durch Hal, oder Ar, substituiert sein kann,
- Hal: F, Cl, Br oder J, und
- A: Alkyl mit 1-8 C-Atomen bedeuten,
sowie deren Salze.

Der Erfindung lag die Aufgabe zugrunde, neue Verbindungen mit wertvollen Eigenschaften aufzufinden, insbesondere solche, die zur Herstellung von Arzneimitteln verwendet werden können.

Es wurde gefunden, daß die Verbindungen der Formel I und ihre Salze sehr wertvolle Eigenschaften besitzen. Vor allem hemmen sie die Aktivität des menschlichen Plasmarenins. Diese Wirkung kann z.B. nach der Methode von F. Fyhrquist et al., Clin.Chem. 22, 250-256 (1976), nachgewiesen werden. Bemerkenswert ist, daß diese Verbindungen sehr spezifische Hemmer des Renins sind; für die Hemmung anderer Aspartylproteinasen (z.B. Pepsin und Kathepsin D) sind in der Regel etwa 100 bis 1000mal so hohe Konzentrationen dieser Verbindungen notwendig wie für die Renin-Hemmung. Die Wirkungen der Verbindungen auf den Blutdruck und/oder auf die Herzfrequenz sowie die Hemmung der Reninaktivität im Blutplasma können ferner an wachen Affen, z.B. weiblichen Affen (Macaca fascicularis) ermittelt werden; dabei können Blutdruck und Herzfrequenz in Anlehnung an die Methode von M.J. Wood et al., J. Hypertension 4, 251-254 (1985) gemessen werden. Zur Stimulierung der Reninaktivität werden die Tiere dabei zweckmäßig mit einem Saluretikum vorbehandelt. Blutproben zur Bestimmung der Plasma-Reninaktivität können durch Punktion der Vena femoralis gewonnen werden.

Die Verbindungen können als Arzneimittelwirkstoffe in der Human- und Veterinärmedizin eingesetzt werden, insbesondere zur Prophylaxe und zur Behandlung von Herz-, Kreislauf- und Gefäßkrankheiten, vor allem von Hypertonie, Herzinsuffizienz und Hyperaldosteronismus. Außerdem können die Verbindungen zu diagnostischen Zwecken verwendet werden, um bei Patienten mit Hypertonie oder Hyperaldosteronismus den möglichen Beitrag der Reninaktivität zur Aufrechterhaltung des pathologischen Zustands zu bestimmen. Solche diagnostische Tests können ausgeführt werden ähnlich wie es in der EP-A-77 028 angegeben ist.

Die vor- und nachstehend aufgeführten Abkürzungen von Aminosäureresten stehen für die Reste -NR'R''-CO-, in der Regel -NH-CHR-CO- (worin R, R' und R'' die für jede Aminosäure bekannte spezifische Bedeutung haben) folgender Aminosäuren:
- βAla: β-Alanin
- Asn: Asparagin
- Asp: Asparaginsäure
- Cal: 3-Cyclohexylalanin
- S-A-Cys: S-Alkyl-cystein [z.B. S-Me-Cys = S-Methyl-cystein
- Gln: Glutamin
- His: Histidin
- Ile: Isoleucin
- Isoser: Isoserin (3-Amino-2-hydroxy-propionsäure)
- Leu: Leucin
- Mal: 3-(p-Methoxyphenyl)-alanin
- Met: Methionin
- Met(O₂): Methionin-S,S-dioxid
- Nle: Norleucin
- Nva: Norvalin (2-Aminovaleriansäure)
- Phe: Phenylalanin
- Ser: Serin
- Thr: Threonin
- Val: Valin.

Ferner bedeutet vor- und nachstehend:
- BOC: tert.-Butoxycarbonyl
- BOM: Benzyloxymethyl
- imi-BOM: Benzyloxymethyl in 1-Stellung des Imidazolrings
- CBZ: Benzyloxycarbonyl
- DCCI: Dicyclohexylcarbodiimid
- DMF: Dimethylformamid
- DNP: 2,4-Dinitrophenyl
- imi-DNP: 2,4-Dinitrophenyl in 1-Stellung des Imidazolrings
- ETOC: Ethoxycarbonyl
- FMOC: 9-Fluorenylmethoxycarbonyl
- HOBt: 1-Hydroxybenzotriazol
- IPOC: Isopropoxycarbonyl
- Pla: den Rest der Phenylmilchsäure -O-CH(CH₂C₆H₅)-CO-(S-Form)
- POA: Phenoxyacetyl
- THF: Tetrahydrofuran.

Sofern die vorstehend genannten Aminosäuren in mehreren enantiomeren Formen auftreten können, so sind vor- und nachstehend, z.B. als Bestandteil der Verbindungen der Formel I, alle diese Formen und auch ihre Gemische (z.B. die DL-Formen) eingeschlossen. Die L-Formen sind bevorzugt. Sofern nachstehend einzelne Verbindungen aufgeführt sind, so beziehen sich die Abkürzungen dieser Aminosäuren jeweils auf die L-Form, sofern nicht ausdrücklich etwas anderes angegeben ist.

Gegenstand der Erfindung ist ferner ein Verfahren zur Herstellung einer Verbindung der Formel I sowie von ihren Salzen, dadurch gekennzeichnet, daß man sie aus einem ihrer funktionellen Derivate durch Behandeln mit einem solvolysierenden oder hydrogenolysierenden Mittel in Freiheit setzt oder daß man eine Carbonsäure der Formel II

R¹-G¹-OH II

worin
G¹
   (a) fehlt,
   (b) Z,
   (c) Z¹ bedeutet
oder eines ihrer reaktionsfähigen Derivate mit einer Aminoverbindung der Formel III

H-G²-NR²-CHR³-CR⁴-(CH₂)ₒ-(CR⁵)ₜ-CE-C_{w}H_{2w}-R⁶ III

worin
G²
   (a) Z bedeutet,
   (b) fehlt,
   (c) Z² bedeutet und

- Z¹ + Z²: zusammen Z bedeuten,
umsetzt,
oder daß man zur Herstellung einer Verbindung der Formel I, worin b = 0 ist, eine Carbonylverbindung der Formel IV

R¹-Z-NR²-CHR³-CR⁴-(CH₂)ₒ-(CR⁵)ₜ-CO-C_{w}H_{2w}-R⁶ IV

mit einem Dithiol der Formel V

HS-(CH₂)₃-SH V

oder mit einem seiner Salze
umsetzt,
oder daß man zur Herstellung einer Verbindung der Formel I, worin E = -SO₂-(CH₂)₃-SO₂- bedeutet, eine Verbindung der Formel VI

R¹-Z-NR²-CHR³-CR⁴(CH₂)ₒ-(CR⁵)ₜ-X VI

worin
- X: Cl, Br, I oder eine reaktionsfähig veresterte OH-Gruppe bedeutet,
mit einer Verbindung der Formel VII

H-CE-C_{w}H_{2w}-R⁶ VII

umsetzt
und daß man gegebenenfalls eine Verbindung der Formel I in eine andere Verbindung der Formel I umwandelt und/oder eine Verbindung der Formel I durch Behandeln mit einer Säure oder Base in eines ihrer Salze überführt.

Vor- und nachstehend haben die Reste bzw. Parameter R¹ bis R⁷, R¹¹, R¹², Z, E, L, T, V, X, a, b, m, n, o, p, s, t, w, y, Ar, Het, Hal, A, G¹, G², Z¹ und Z² die bei den Formeln I, II oder III angegebenen Bedeutungen, falls nicht ausdrücklich etwas anderes angegeben ist.

Die Verbindungen der Formel I und auch die Ausgangsstoffe zu ihrer Herstellung werden im übrigen nach an sich bekannten Methoden hergestellt, wie sie in der Literatur (z.B. in den Standardwerken wie Houben-Weyl, Methoden der organischen Chemie, Georg-Thieme-Verlag, Stuttgart; ferner EP-A-45665, EP-A-77028, EP-A-77029, EP-A-81783, EP-A-249096) beschrieben sind, und zwar unter Reaktionsbedingungen, die für die genannten Umsetzungen bekannt und geeignet sind. Dabei kann man auch von an sich bekannten, hier nicht näher erwähnten Varianten Gebrauch machen.

Die Ausgangsstoffe können, falls erwünscht, auch in situ gebildet werden, so daß man sie aus dem Reaktionsgemisch nicht isoliert, sondern sofort weiter zu den Verbindungen der Formel I umsetzt.

Die Verbindungen der Formel I können erhalten werden, indem man sie aus ihren funktionellen Derivaten durch Solvolyse, insbesondere Hydrolyse, oder durch Hydrogenolyse in Freiheit setzt.

Bevorzugte Ausgangsstoffe für die Solvolyse bzw. Hydrogenolyse sind solche, die sonst der Formel I entsprechen, aber an Stelle einer oder mehrerer freier Amino- und/oder Hydroxygruppen entsprechende geschützte Amino- und/oder Hydroxygruppen enthalten, vorzugsweise solche, die an Stelle eines H-Atoms, das mit einem N-Atom verbunden ist, eine Aminoschutzgruppe tragen, z.B. solche die der Formel I entsprechen, aber an Stelle einer His-Gruppe eine N(im)-R'-His-Gruppe (worin R' eine Aminoschutzgruppe bedeutet, z.B. BOM oder DNP) enthalten.

Ferner sind Ausgangsstoffe bevorzugt, die an Stelle des H-Atoms einer Hydroxygruppe eine Hydroxyschutzgruppe tragen, z.B. solche der Formel R¹-Z-NR²-CHR³-CHOR''-(CH₂)ₒ-(CR⁵)ₜ- -CE-C_{w}H_{2w}-R⁶, worin R'' eine Hydroxyschutzgruppe bedeutet.

Es können auch mehrere - gleiche oder verschiedene - geschützte Amino- und/oder Hydroxygruppen im Molekül des Ausgangsstoffes vorhanden sein. Falls die vorhandenen Schutzgruppen voneinander verschieden sind, können sie auch selektiv abgespalten werden.

Der Ausdruck "Aminoschutzgruppe" ist allgemein bekannt und bezieht sich auf Gruppen, die geeignet sind, eine Aminogruppe vor chemischen Umsetzungen zu schützen (zu blockieren), die aber leicht entfernbar sind, nachdem die gewünschte chemische Reaktion an einer anderen Stelle des Moleküls durchgeführt worden ist. Typisch für solche Gruppen sind insbesondere unsubstituierte oder substituierte Acyl-, Aryl- (z.B. DNP), Aralkoxymethyl- (z.B. BOM) oder Aralkylgruppen (z.B. Benzyl, 4-Nitrobenzyl, Triphenylmethyl). Da die Aminoschutzgruppen nach der gewünschten Reaktion (oder Reaktionsfolge) entfernt werden, ist ihre Art und Größe im übrigen nicht kritisch; bevorzugt werden jedoch solche mit 1-20, insbesondere 1-8 C-Atomen. Der Ausdruck "Acylgruppe" ist im Zusammenhang mit dem vorliegenden Verfahren im weitesten Sinne aufzufassen. Er umschließt von aliphatischen, araliphatischen, aromatischen oder heterocyclischen Carbonsäuren oder Sulfonsäuren abgeleitete Acylgruppen sowie insbesondere Alkoxycarbonyl-, Aryloxycarbonyl- und vor allem Aralkoxycarbonylgruppen. Beispiele für derartige Acylgruppen sind Alkanoyl wie Acetyl, Propionyl, Butyryl; Aralkanoyl wie Phenylacetyl; Aroyl wie Benzoyl oder Toluyl; Aryloxyalkanoyl wie POA; Alkoxycarbonyl wie Methoxycarbonyl, ETOC, 2,2,2-Trichlorethoxycarbonyl, IPOC, BOC, 2-Jodethoxycarbonyl; Aralkyloxycarbonyl wie CBZ, 4-Methoxybenzyloxycarbonyl, FMOC. Bevorzugte Aminoschutzgruppen sind BOC, DNP und BOM, ferner CBZ, FMOC, Benzyl und Acetyl.

Der Ausdruck "Hydroxyschutzgruppe" ist ebenfalls allgemein bekannt und bezieht sich auf Gruppen, die geeignet sind, eine Hydroxygruppe vor chemischen Umsetzungen zu schützen, die aber leicht entfernbar sind, nachdem die gewünschte chemische Reaktion an einer anderen Stelle des Moleküls durchgeführt worden ist. Typisch für solche Gruppen sind die oben genannten unsubstituierten oder substituierten Aryl-, Aralkyl- oder Acylgruppen, ferner auch Alkylgruppen. Die Natur und Größe der Hydroxyschutzgruppen ist nicht kritisch, da sie nach der gewünschten chemischen Reaktion oder Reaktionsfolge wieder entfernt werden; bevorzugt sind Gruppen mit 1-20, insbesondere 1-10 C-Atomen. Beispiele für Hydroxyschutzgruppen sind u.a. tert.-Butyl, Benzyl, p-Nitrobenzoyl, p-Toluolsulfonyl und Acetyl, wobei Benzyl und Acetyl besonders bevorzugt sind.

Die als Ausgangsstoffe zu verwendenden funktionellen Derivate der Verbindungen der Formel I können nach üblichen Methoden der Aminosäure- und Peptidsynthese hergestellt werden, wie sie z.B. in den genannten Standardwerken und Patentanmeldungen beschrieben sind, z.B. auch nach der Festphasenmethode nach Merrifield.

Das In-Freiheit-Setzen der Verbindungen der Formel I aus ihren funktionellen Derivaten gelingt - je nach der benutzten Schutzgruppe - z.B. mit starken Säuren, zweckmäßig mit Trifluoressigsäure oder Perchlorsäure, aber auch mit anderen starken anorganischen Säuren wie Salzsäure oder Schwefelsäure, starken organischen Carbonsäuren wie Trichloressigsäure oder Sulfonsäuren wie Benzol- oder p-Toluolsulfonsäure. Die Anwesenheit eines zusätzlichen inerten Lösungsmittels ist möglich, aber nicht immer erforderlich. Als inerte Lösungsmittel eignen sich vorzugsweise organische, beispielsweise Carbonsäuren wie Essigsäure, Ether wie Tetrahydrofuran oder Dioxan, Amide wie DMF, halogenierte Kohlenwasserstoffe wie Dichlormethan, ferner auch Alkohole wie Methanol, Ethanol oder Isopropanol sowie Wasser. Ferner kommen Gemische der vorgenannten Lösungsmittel in Frage. Trifluoressigsäure wird vorzugsweise im Überschuß ohne Zusatz eines weiteren Lösungsmittels verwendet, Perchlorsäure in Form eines Gemisches aus Essigsäure und 70%iger Perchlorsäure im Verhältnis 9:1. Die Reaktionstemperaturen für die Spaltung liegen zweckmäßig zwischen etwa 0 und etwa 50°, vorzugsweise arbeitet man zwischen 15 und 30° (Raumtemperatur).

Die BOC-Gruppe kann z.B. bevorzugt mit 40%iger Trifluoressigsäure in Dichlormethan oder mit etwa 3 bis 5 n HCl in Ethylacetat oder Dioxan bei 15-30° abgespalten weden, die FMOC-Gruppe mit einer etwa 5-20%igen Lösung von Dimethylamin, Diethylamin oder Piperidin in DMF bei 15-30°. Eine Abspaltung der DNP-Gruppe gelingt z.B. auch mit einer etwa 3-10%igen Lösung von 2-Mercaptoethanol in DMF/Wasser bei 15-30°.

Hydrogenolytisch entfernbare Schutzgruppen (z.B. BOM, CBZ oder Benzyl) können z.B. durch Behandeln mit Wasserstoff in Gegenwart eines Katalysators (z.B. eines Edelmetallkatalysators wie Palladium, zweckmäßig auf einem Träger wie Kohle) abgespalten werden. Als Lösungsmittel eignen sich dabei die oben angegebenen, insbesondere z.B. Alkohole wie Methanol oder Ethanol oder Amide wie DMF. Die Hydrogenolyse wird in der Regel bei Temperaturen zwischen etwa 0 und 100° und Drucken zwischen etwa 1 und 200 bar, bevorzugt bei 20-30° und 1-10 bar durchgeführt. Eine Hydrogenolyse der CBZ-Gruppe gelingt z.B. gut an 5-10%igem Pd-C in Methanol bei 20-30°.

Verbindungen der Formel I können auch durch direkte Peptidsynthese aus einer Carbonsäure- (Formel II) und einer Aminkomponente (Formel III) erhalten werden. Als Carbonsäurekomponenten eignen sich z.B. solche der Teilformeln (a) R¹-OH, (b) R¹-Z-OH, als Aminkomponenten solche der Teilformeln (a) H-Z-NR²-CHR³-CR⁴-(CH₂)ₒ-(CR⁵)ₜ-CE-C_{w}H_{2w}-R⁶ (b) H-NR²-CHR³-CR⁴-(CH₂)ₒ-(CR⁵)ₜ-CE-C_{w}H_{2w}-R⁶. Die Peptidbindung kann aber auch innerhalb der Gruppe Z geknüpft werden; dabei wird eine Carbonsäure der Formel R¹-Z¹-OH mit einer Aminoverbindung der Formel H-Z²-NR²-CHR³-CR⁴-(CH₂)ₒ-(CR⁵)ₜ-CE-C_{w}H_{2w}-R⁶ umgesetzt, wobei Z¹ + Z² = Z ist. Dabei arbeitet man zweckmäßig nach üblichen Methoden der Peptid-Synthese, wie sie z.B. in Houben-Weyl, 1.c., Band 15/II, Seiten 1-806 (1974) beschrieben sind.

Die Reaktion gelingt vorzugsweise in Gegenwart eines Dehydratisierungsmittels, z.B. eines Carbodiimids wie DCCI oder Dimethylaminopropylethyl-carbodiimid, ferner Propanphosphonsäureanhydrid (vgl. Angew.Chem. 92, 129 (1980)), Diphenylphosphorylazid oder 2-Ethoxy-N-ethoxycarbonyl-1,2-dihydrochinolin, in einem inerten Lösungsmittel, z.B. einem halogenierten Kohlenwasserstoff wie Dichlormethan, einem Ether wie THF oder Dioxan, einem Amid wie DMF oder Dimethylacetamid, einem Nitril wie Acetonitril, bei Temperaturen zwischen etwa -10 und 40, vorzugsweise zwischen 0 und 30°.

An Stelle von II bzw. III können auch geeignete reaktionsfähige Derivate dieser Stoffe in die Reaktion eingesetzt werden, z.B. solche, in denen reaktive Gruppen intermediär durch Schutzgruppen blockiert sind. Die Säurederivate II können z.B. in Form ihrer aktivierten Ester verwendet werden, die zweckmäßig in situ gebildet werden, z.B. durch Zusatz von HOBt oder N-Hydroxysuccinimid.

Harnstoffderivate der Formel I [R¹ = R⁸-NH-CO- oder R¹¹-CₘH₂ₘ-(T)ₛ-(V)_{y}-CₙH₂ₙ-NH-CO-] sind z.B erhältlich, indem man ein entsprechendes Isocyanat mit einem Amin der Formel H-Z-NR²-CHR³-CR⁴-(CH₂)ₒ-(CR⁵)ₜ-CE-C_{w}H_{2w}-R⁶ (IIa) umsetzt, zweckmäßig in einem inerten Lösungsmittel wie THF bei Temperaturen zwischen etwa -10 und 40°, vorzugsweise zwischen 10 und 30°.

Die Ausgangsstoffe der Formeln II und III sind großenteils bekannt. Sofern sie nicht bekannt sind, können sie nach bekannten Methoden, z.B. den oben angegebenen Methoden der Peptidsynthese und der Abspaltung von Schutzgruppen hergestellt werden.

Zur Herstellung einer Verbindung der Formel I (eines Thioketals), worin b = 0 ist, kann man ferner eine Carbonylverbindung der Formel IV mit einem Dithiol der Formel V (1,3-Propandithiol) oder mit einem seiner Salze, vorzugsweise dem Di-Na- oder Di-K-Salz umsetzen, zweckmäßig in einem der angegebenen inerten Lösungsmittel, z.B. einem halogenierten Kohlenwasserstoff wie Dichlormethan, in Gegenwart eines Katalysators, z.B. einer Lewis-Säure wie BF₃-Etherat, bei Temperaturen zwischen etwa -10 und 60°, vorzugsweise zwischen -5 und 10°.

Zur Herstellung eines Disulfons der Formel I, worin E -SO₂-(CH₂)₃-SO₂- bedeutet, kann man eine Verbindung der Formel VI mit einer Verbindung der Formel VII unter Alkylierungsbedingungen umsetzen. In den Verbindungen der Formel VI ist X vorzugsweise Cl, Br oder I; falls X eine reaktionsfähig veresterte OH-Gruppe bedeutet, so ist es vorzugsweise Alkylsulfonyloxy mit 1-6 C-Atomen, z.B. Methansulfonyloxy, oder Arylsulfonyloxy mit 6-10 C-Atomen, z.B. Benzol-, p-Toluol- oder 1- oder 2-Naphthalinsulfonyloxy. Bei der Umsetzung von VI mit VII wird zweckmäßig zunächst das reaktive Anion von VII durch Reaktion mit einer starken Base, z.B. NaH, in einem inerten Lösungsmittel, z.B. einem Amid wie DMF, hergestellt; hierauf fügt man VI hinzu und läßt bei Temperaturen zwischen etwa 10 und 60° ausreagieren.

Die Ausgangsstoffe der Formeln IV, V, VI und VII sind teilweise bekannt. Falls sie nicht bekannt sind, können sie nach bekannten Methoden in Analogie zu bekannten Stoffen hergestellt werden.

Es ist ferner möglich, eine Verbindung der Formel I in eine andere Verbindung der Formel I umzuwandeln, z.B. indem man freie Amino- und/oder Hydroxygruppen funktionell abwandelt und/oder Thioethergruppen zu Sulfongruppen oxydiert und/oder funktionell abgewandelte Amino- und/oder Hydroxygruppen durch Solvolyse oder Hydrogenolyse freisetzt und/oder reaktive CH-Gruppen alkyliert.

Verbindungen der Formel I, worin R² H und R⁴ OH bedeutet, können durch Reaktion mit Carbonylverbindungen der Formel A-CO-A oder deren funktionellen Derivaten (z.B. mit Ketalen wie 2,2-Dimethoxypropan) in cyclische Aminale (Oxazolidine) der Formel I, worin R⁴ (H, R¹²) und R² und R¹² zusammen -C(A₂)-O- bedeuten, umgewandelt werden, zweckmäßig in Gegenwart eines inerten Lösungsmittels wie Toluol in Gegenwart eines Dehydratisierungskatalysators wie p-Toluolsulfonsäure bei Temperaturen zwischen 30 und 120°.

Thioethergruppen, insbesondere im Rest E, können zu Sulfongruppen oxydiert werden, zweckmäßig mit einem Oxydationsmittel wie H₂O₂ oder einer Persäure, z.B. 3-Chlorperbenzoesäure oder Monoperoxyphthalsäure, oder einem ihrer Salze in einem inerten Lösungsmittel wie Dichlormethan oder THF bei Temperaturen zwischen etwa 0 und 40°.

Gewünschtenfalls kann in einer Verbindung der Formel I eine funktionell abgewandelte Amino- und/oder Hydroxygruppe durch Solvolyse oder Hydrogenolyse nach einer der oben beschriebenen Methoden in Freiheit gesetzt werden.

So kann z. B. eine Verbindung der Formel I, die eine NH-CO-O-A-Gruppe enthält, in die entsprechende Verbindung der Formel I umgewandelt werden, die stattdessen eine H₂N-Gruppe enthält, zweckmäßig durch selektive Solvolyse nach einer der oben angegebenen Methoden. AOOC-Gruppen können z.B. mit NaOH oder KOH in Wasser-Dioxan bei Temperaturen zwischen 0 und 40°, vorzugsweise 10 und 30°, verseift werden.

Es ist ferner möglich, reaktive CH-Gruppen zu alkylieren, z.B. Verbindungen der Formel I, worin w = 0, R⁶ = H und b = 2 ist, mit Verbindungen der Formel R⁶-C_{w}H_{2w}-X, worin R⁶ von H und/oder w von 0 verschieden ist, umzusetzen, zweckmäßig unter ähnlichen Bedingungen, wie sie oben für die Reaktion von VI mit VII angegeben sind.

Eine Base der Formel I kann mit einer Säure in das zugehörige Säureadditionssalz übergeführt werden. Für diese Umsetzung kommen insbesondere Säuren in Frage, die physiologisch unbedenkliche Salze liefern, So können anorganische Säuren verwendet werden, z.B. Schwefelsäure, Salpetersäure, Halogenwasserstoffsäuren wie Chlorwasserstoffsäure oder Bromwasserstoffsäure, Phosphorsäuren wie Orthophosphorsäure, Sulfaminsäure, ferner organische Säuren, insbesondere aliphatische, alicyclische, araliphatische, aromatische oder heterocyclische ein- oder mehrbasige Carbon-, Sulfon- oder Schwefelsäuren, z.B. Ameisensäure, Essigsäure, Propionsäure, Pivalinsäure, Diethylessigsäure, Malonsäure, Bernsteinsäure, Pimelinsäure, Fumarsäure, Maleinsäure, Milchsäure, Weinsäure, Äpfelsäure, Citronensäure, Gluconsäure, Ascorbinsäure, Nicotinsäure, Isonicotinsäure, Methan- oder Ethansulfonsäure, Ethandisulfonsäure, 2-Hydroxyethansulfonsäure, Benzolsulfonsäure, p-Toluolsulfonsäure, Naphthalin-mono- und -disulfonsäuren, Laurylschwefelsäure. Salze mit physiologisch nicht unbedenklichen Säuren, z.B. Pikrate, können zur Isolierung und/oder Aufreinigung der Verbindungen der Formel I verwendet werden.

Die neuen Verbindungen der Formel I und ihre physiologisch unbedenklichen Salze können zur Herstellung pharmazeutischer Präparate verwendet werden, indem man sie zusammen mit mindestens einem Träger- oder Hilfsstoff und, falls erwünscht, zusammen mit einem oder mehreren weiteren Wirkstoff(en) in eine geeignete Dosierungsform bringt. Die so erhaltenen Zubereitungen können als Arzneimittel in der Human- oder Veterinärmedizin eingesetzt werden. Als Trägersubstanzen kommen organische oder anorganische Stoffe in Frage, die sich für die enterale (z.B. orale oder rektale) oder parenterale Applikation oder für eine Applikation in Form eines Inhalations-Sprays eignen und mit den neuen Verbindungen nicht reagieren, beispielsweise Wasser, pflanzliche Öle, Benzylalkohole, Polyethylenglykole, Glycerintriacetat und andere Fettsäureglyceride, Gelatine, Sojalecithin, Kohlehydrate wie Lactose oder Stärke, Magnesiumstearat, Talk, Cellulose. Zur oralen Anwendung dienen insbesondere Tabletten, Dragees, Kapseln, Sirupe, Säfte oder Tropfen; von Interesse sind speziell Lacktabletten und Kapseln mit magensaftresistenten Überzügen bzw. Kapselhüllen. Zur rektalen Anwendung dienen Suppositorien, zur parenteralen Applikation Lösungen, vorzugsweise ölige oder wässerige Lösungen, ferner Suspensionen, Emulsionen oder Implantate. Für die Applikation als Inhalations-Spray können Sprays verwendet werden, die den Wirkstoff entweder gelöst oder suspendiert in einem Treibgasgemisch (z.B. Fluorchlorkohlenwasserstoffen) enthalten. Zweckmäßig verwendet man den Wirkstoff dabei in mikronisierter Form, wobei ein oder mehrere zusätzliche physiologisch verträgliche Lösungsmittel zugegen sein können, z.B. Ethanol. Inhalationslösungen können mit Hilfe üblicher Inhalatoren verabfolgt werden. Die neuen Verbindungen können auch lyophilisiert und die erhaltenen Lyophilisate z.B. zur Herstellung von Injektionspräparaten verwendet werden. Die angegebenen Zubereitungen können sterilisiert sein und/oder Hilfsstoffe wie Konservierungs-, Stabilisierungs- und/oder Netzmittel, Emulgatoren, Salze zur Beeinflussung des osmotischen Druckes, Puffersubstanzen, Farb- und/oder Aromastoffe enthalten. Sie können, falls erwünscht, auch einen oder mehrere weitere Wirkstoffe enthalten, z.B. ein oder mehrere Vitamine.

Die erfindungsgemäßen Substanzen werden in der Regel in Analogie zu anderen bekannten, im Handel befindlichen Peptiden, insbesondere aber in Analogie zu den in der EP-A-249096 beschriebenen Verbindungen verabreicht, vorzugsweise in Dosierungen zwischen etwa 10 mg und 1 g, insbesondere zwischen 50 und 500 mg pro Dosierungseinheit. Die tägliche Dosierung liegt vorzugsweise zwischen etwa 0,2 und 20 mg/kg, insbesondere 1 und 10 mg/kg Körpergewicht. Die spezielle Dosis für jeden bestimmten Patienten hängt jedoch von den verschiedensten Faktoren ab, beispielsweise von der Wirksamkeit der eingesetzten speziellen Verbindung, vom Alter, Körpergewicht, allgemeinen Gesundheitszustand, Geschlecht, von der Kost, vom Verabfolgungszeitpunkt und -weg, von der Ausscheidungsgeschwindigkeit, Arzneistoffkombination und Schwere der jeweiligen Erkrankung, welcher die Therapie gilt. Die parenterale Applikation ist bevorzugt.

Renin-abhängige Hypertension und Hyperaldosteronismus sowie ferner auch durch Retroviren verursachte Erkrankungen, insbesondere AIDS ("acquired immunodeficiency syndrome") können wirksam behandelt werden durch Verabfolgung von Dosierungen zwischen insbesondere etwa 1 und 300, vorzugsweise zwischen 5 und 50 mg/kg Körpergewicht. Für diagnostische Zwecke können die neuen Verbindungen zweckmäßig in Einzeldosen zwischen etwa 0,1 und 10 mg/kg Körpergewicht verabreicht werden.

Vor- und nachstehend sind alle Temperaturen in °C angegeben. In den nachfolgenden Beispielen bedeutet "übliche Aufarbeitung": Man gibt, falls erforderlich, Wasser hinzu, stellt je nach Konstitution des Endprodukts auf pH-Werte zwischen 2 und 8 ein, extrahiert mit Ethylacetat oder Dichlormethan, trennt ab, trocknet die organische Phase über Natriumsulfat, dampft ein und reinigt durch Chromatographie an Kieselgel und/oder Kristallisation. MS = Massenspektrum; FAB = Massenspektrum nach der Fast-Atom-Bombardment-Methode.

### Beispiel 1

Ein Gemisch von 0,66 g 2-[(2S,3S)-3-BOC-Phe-(imi-DNP-His)-amino-4-cyclohexyl-2-hydroxy-butyl]-1,3-dithian [FAB: 840, Diastereomerengemisch; erhältlich aus 2-[(2S,3S)-3-Amino-4-cyclohexyl-2-hydroxy-butyl]-1,3-dithian (vgl. Beispiel 11) und BOC-Phe-(imi-DNP-His)-OH nach der Methode von Beispiel 3], 15 ml DMF, 3 ml Wasser und 0,3 ml 5%iger NaHCO₃-Lösung wird mit 0,22 g 2-Mercaptoethanol versetzt und 17 Std. bei 20° stehengelassen. Übliche Aufarbeitung (Kieselgel, Dichlormethan/Methanol mit NH₃ gesättigt) liefert 2 Diastereomere von 2-[(2S,3S)-3-BOC-Phe-His-amino-4-cyclohexyl-2-hydroxy-butyl]-1,3-dithian; F. 140-145° (FAB 674) bzw. F. 90-92° (Zers.; FAB 674).

Analog erhält man aus den entsprechenden imi-DNP-His-Derivaten die nachstehenden 1,3-Dithian-1,1,3,3-tetroxide:
2-[(2S,3S)-3-BOC-Phe-His-amino-4-cyclohexyl-2-hydroxy-butyl]-, F 231-232° (Zers.); FAB 738; erhältlich über 2-[(2S,3S)-3-BOC-(imi-DNP-His)-amino-4-cyclohexyl-2-hydroxy-butyl]-1,3-dithian-1,1,3,3-tetroxid [F. 188-190° (Zers.)] und 2-[(2S,3S)-3-H-(imi-DNP-His)-amino-4-cyclohexyl-2-hydroxybutyl]-1,3-dithian-1,1,3,3-tetroxid [F. 203° (Zers.)]
2-[(2S,3S)-3-BOC-Phe-His-amino-4-cyclohexyl-2-hydroxy-butyl]-2-methyl-, F. 145° (Zers.); FAB 752; erhältlich über 2-[(2S,3S)-3-BOC-(imi-DNP-His)-amino-4-cyclohexyl-2-hydroxy-butyl]-2-methyl-1,3-dithian-1,1,3,3-tetroxid [F. 125-130° (Zers.); FAB 771] und 2-[(2S,3S)-3-H-(imi-DNP-His)-amino-4-cyclohexyl-2-hydroxy-butyl]-2-methyl-1,3-dithian-1,1,3,3-tetroxid [F. 228° (Zers.)]
2-[(2S,3S)-3-(4-BOC-amino-piperidinocarbonyl-Phe-His-amino)-4-cyclohexyl-2-hydroxy-butyl]-, FAB 865
2-[(2S,3S)-3-(4-BOC-amino-piperidinocarbonyl-Phe-His-amino)-4-cyclohexyl-2-hydroxy-butyl]-2-methyl-, FAB 879
2-[(2S,3S)-3-(3-BOC-amino-3-methyl-butyryl-Mal-His-amino)-4-cyclohexyl-2-hydroxy-butyl]-, FAB 867
2-[(2S,3S)-3-(3-BOC-amino-3-methyl-butyryl-Mal-His-amino)-4-cyclohexyl-2-hydroxy-butyl]-2-methyl-, FAB 881
2-[(2S,3S)-3-(2-Benzyl-3-tert.-butylsulfonyl-propionyl-His-amino)-4-cyclohexyl-2-hydroxy-butyl]-, 2 Isomere, FAB jeweils 757; eines der Isomeren gibt ein Hydrochlorid, F. 175-180 ° (Zers.)
2-[(2S,3S)-3-(2-Benzyl-3-tert.-butylsulfonyl-propionyl-His-amino)-4-cyclohexyl-2-hydroxy-butyl]-2-methyl-, 2 Isomere, FAB jeweils 771
2-[(2S,3S)-3-BOC-Mal-His-amino-4-cyclohexyl-2-hydroxy-butyl]-
2-[(2S,3S)-3-Morpholinocarbonyl-Phe-His-amino-4-cyclohexyl-2-hydroxy-butyl]-
2-[(3S,4S)-4-BOC-Phe-His-amino-5-cyclohexyl-3-hydroxy-pentyl]-, F. 170-174° (Zers.), FAB 753; erhältlich über
2-[(3S,4S)-4-BOC-(imi-DNP-His)-amino-5-cyclohexyl-3-hydroxy-pentyl]-1,3-dithian-1,1,3,3-tetroxid (F. 132-135°; FAB 771) und 2-[(3S,4S)-4-H-(imi-DNP-His)-amino-5-cyclohexyl-3-hydroxy-pentyl]-1,3-dithian-1,1,3,3-tetroxid [F. 205-210° (Zers.); FAB 671]
2-[(3S,4S)-4-BOC-Phe-His-amino-5-cyclohexyl-3-hydroxy-pentyl]-2-methyl-, F. 118-122° (Zers.), FAB 766; erhältlich über 2-[(3S,4S)-4-BOC-(imi-DNP-His)-amino-5-cyclohexyl-3-hydroxy-pentyl]-2-methyl-1,3-dithian-1,1,3,3-tetroxid, [F. 136-140° (Zers.); FAB 785] und
2-[(3S,4S)-4-H-(imi-DNP-His)-amino-5-cyclohexyl-3-hydroxy-pentyl]-2-methyl-1,3-dithian-1,1,3,3-tetroxid, [F. 211-214° (Zers.); FAB 685]
2-[(3S,4S)-4-(4-BOC-amino-piperidinocarbonyl)-Phe-His-amino-5-cyclohexyl-3-hydroxy-pentyl]-, FAB 878
2-[(3S,4S)-4-(4-BOC-amino-piperidinocarbonyl)-Phe-His-amino-5-cyclohexyl-3-hydroxy-pentyl]-2-methyl-, F. 158-162° (Zers.), FAB 892
2-[(3S,4S)-4-(3-BOC-amino-3-methyl-butyryl-Mal-His-amino)-5-cyclohexyl-3-hydroxy-pentyl]-, F. 130-135° (Zers.), FAB 881
2-[(3S,4S)-4-(3-BOC-amino-3-methyl-butyryl-Mal-His-amino)-5-cyclohexyl-3-hydroxy-pentyl]-2-methyl-, F. 122-126° (Zers.); FAB 895
2-[(3S,4S)-4-(2-Benzyl-3-tert.-butylsulfonyl-propionyl-His-amino)-5-cyclohexyl-3-hydroxy-pentyl]-, F. 230° (Zers.); FAB 771
2-[(3S,4S)-4-(2-Benzyl-3-tert.-butylsulfonyl-propionyl-His-amino)-5-cyclohexyl-3-hydroxy-pentyl]-2-methyl-.

### Beispiel 2

Man löst 1 g 2-[(2S,3S)-3-BOC-Phe-(imi-BOM-His)-amino-4-cyclohexyl-2-hydroxybutyl]-1,3-dithian-1,1,3,3-tetroxid [erhältlich aus 2-[(2S,3S)-3-Amino-4-cyclohexyl-2-hydroxybutyl]-1,3-dithian-1,1,3,3-tetroxid (vgl. Beispiel 12) und BOC-Phe-(imi-BOM-His)-OH nach der Methode von Beispiel 3] in 25 ml Ethanol, hydriert an 0,4 g 10%ig. Pd-C bei 20° und 1 bar bis zum Ende der H₂-Aufnahme, filtriert, dampft ein und erhält nach chromatographischer Reinigung 2-[(2S,3S)-3-BOC-Phe-His-amino-4-cyclohexyl-2-hydroxybutyl]-1,3-dithian-1,1,3,3-tetroxid; F. 231-232°; FAB 738.

### Beispiel 3

Eine Lösung von 3,21 g 2-[(2S,3S)-3-Amino-4-cyclohexyl-2-hydroxy-butyl]-1,3-dithian-1,1,3,3-tetroxid in 60 ml Dichlormethan wird mit 1,01 g N-Methylmorpholin versetzt. Unter Rühren gibt man 2,31 g BOC-Leu-OH, 1,35 g HOBt und eine Lösung von 2,06 g DCCI in 50 ml Dichlormethan hinzu, rührt 14 Std. bei 2-6°, filtriert den ausgeschiedenen Dicyclohexylharnstoff ab und dampft das Filtrat ein. Nach üblicher Aufarbeitung erhält man 2-[(2S,3S)-3-BOC-Leu-amino-4-cyclohexyl-2-hydroxy-butyl]-1,3-dithian-1,1,3,3-tetroxid, F. 228°; FAB 567.

Analog erhält man die folgenden 1,3-Dithian-1,1,3,3-tetroxide:
2-[(2S,3S)-3-BOC-Phe―amino-4-cyclohexyl-2-hydroxy-butyl]-
2-[(2S,3S)-3-BOC-Phe-βAla-amino-4-cyclohexyl-2-hydroxy-butyl]-
2-[(2S,3S)-3-BOC-Phe-Ile-amino-4-cyclohexyl-2-hydroxy-butyl]-
2-[(2S,3S)-3-BOC-Phe-Leu-amino-4-cyclohexyl-2-hydroxy-butyl]-
2-[(2S,3S)-3-BOC-Phe-Nle-amino-4-cyclohexyl-2-hydroxy-butyl]-
2-[(2S,3S)-3-BOC-Phe-Nva-amino-4-cyclohexyl-2-hydroxy-butyl]-
2-[(2S,3S)-3-BOC-Phe-Val-amino-4-cyclohexyl-2-hydroxy-butyl]-
2-[(2S,3S)-3-BOC-Mal―amino-4-cyclohexyl-2-hydroxy-butyl]-
2-[(2S,3S)-3-BOC-Mal-βAla-amino-4-cyclohexyl-2-hydroxy-butyl]-
2-[(2S,3S)-3-BOC-Mal-Ile-amino-4-cyclohexyl-2-hydroxy-butyl]-
2-[(2S,3S)-3-BOC-Mal-Leu-amino-4-cyclohexyl-2-hydroxy-butyl]-
2-[(2S,3S)-3-BOC-Mal-Nle-amino-4-cyclohexyl-2-hydroxy-butyl]-
2-[(2S,3S)-3-BOC-Mal-Nva-amino-4-cyclohexyl-2-hydroxy-butyl]-
2-[(2S,3S)-3-BOC-Mal-Val-amino-4-cyclohexyl-2-hydroxy-butyl]-
2-[(2S,3S)-3-(4-BOC-amino-piperidinocarbonyl-Phe-amino)-4-cyclohexyl-2-hydroxybutyl]-, F. 185-190°, FAB 727
2-[(2S,3S)-3-(4-BOC-amino-piperidinocarbonyl-Phe-βAla-amino)-4-cyclohexyl-2-hydroxybutyl]-, F. 133-135°, FAB 798
2-[(2S,3S)-3-(4-BOC-amino-piperidinocarbonyl-Phe-Ile-amino)-4-cyclohexyl-2-hydroxybutyl]-
2-[(2S,3S)-3-(4-BOC-amino-piperidinocarbonyl-Phe-Leu-amino)-4-cyclohexyl-2-hydroxybutyl]-, FAB 840
2-[(2S,3S)-3-(4-BOC-amino-piperidinocarbonyl-Phe-Nle-amino)-4-cyclohexyl-2-hydroxybutyl]-, F. 156-163°, FAB 840
2-[(2S,3S)-3-(4-BOC-amino-piperidinocarbonyl-Phe-Nva-amino)-4-cyclohexyl-2-hydroxybutyl]-, F. 154-157°, FAB 826
2-[(2S,3S)-3-(4-BOC-amino-piperidinocarbonyl-Phe-Val-amino)-4-cyclohexyl-2-hydroxybutyl]-, F. 185-188°, FAB 826
2-[(2S,3S)-3-(Morpholinocarbonyl-Phe-amino)-4-cyclo-hexyl-2-hydroxy-butyl]-
2-[(2S,3S)-3-(Morpholinocarbonyl-Phe-βAla-amino)-4-cyclohexyl-2-hydroxy-butyl]-
2-[(2S,3S)-3-(Morpholinocarbonyl-Phe-Ile-amino)-4-cyclohexyl-2-hydroxy-butyl]-
2-[(2S,3S)-3-(Morpholinocarbonyl-Phe-Leu-amino)-4-cyclohexyl-2-hydroxy-butyl]-
2-[(2S,3S)-3-(Morpholinocarbonyl-Phe-Nle-amino)-4-cyclohexyl-2-hydroxy-butyl]-
2-[(2S,3S)-3-(Morpholinocarbonyl-Phe-Nva-amino)-4-cyclohexyl-2-hydroxy-butyl]-
2-[(2S,3S)-3-(Morpholinocarbonyl-Phe-Val-amino)-4-cyclohexyl-2-hydroxy-butyl]-.

### Beispiel 4

Analog Beispiel 3 erhält man aus 3-BOC-amino-3-methyl-buttersäure und 2-[(2S,3S)-4-Cyclohexyl-2-hydroxy-3-(H-Phe-βAla-amino)-butyl]-1,3-dithian-1,1,3,3-tetroxid das 2-[(2S,3S)-3-(3-BOC-amino-3-methyl-butyryl-Phe-βAla-amino)-butyl]-1,3-dithian-1,1,3,3-tetroxid.

### Beispiel 5

Analog Beispiel 3 erhält man aus N-(4-BOC-amino-piperidinocarbonyl)-Phe-OH und 2-[(2S,3S)-4-Cyclohexyl-2-hydroxy-3-(H-Leu-amino)-butyl]-1,3-dithian-1,1,3,3-tetroxid (Beispiel 11) das 2-[(2S,3S)-3-(4-BOC-amino-piperidinocarbonyl-Phe-Leu-amino)-4-cyclohexyl-2-hydroxy-butyl]-1,3-dithian-1,1,3,3-tetroxid, FAB 840.

### Beispiel 6

Ein Gemisch von 1,02 g 3-BOC-4-cyclohexylmethyl-2,2-dimethyl-5-oxazolidinyl-acetaldehyd in 30 ml CH₂Cl₂ wird bei 0° mit 0,37 g 1,3-Propandithiol und 0,13 ml BF₃-Etherat versetzt. Nach Waschen mit NaHCO₃-Lösung dampft man ein und erhält nach Chromatographie (Kieselgel, CH₂Cl₂/Ether 95:5) 2-[(2S,3S)-3-BOC-amino-4-cyclohexyl-2-hydroxy-butyl]-1,3-dithian, farbloses Harz, MS: 389.

### Beispiel 7

Eine Lösung von 0,9 g 1,3-Dithian-1,1,3,3-tetroxid in 10 ml DMF wird unter Rühren bei 20° und 0,15 g 80%igem NaH versetzt. Nach 30 Min. werden 2,1 g 2-[(4S,5S)-3-BOC-4-cyclohexylmethyl-2,2-dimethyl-5-oxazolidinyl]-ethanol-methansulfonsäureester hinzugefügt. Man rührt 19 Std. bei 50°, verdünnt mit Wasser, filtriert das erhaltene 2-[2-((4S,5S)-3-BOC-4-cyclohexylmethyl-2,2-dimethyl-5-oxazolidinyl)-ethyl]-1,3-dithian-1,1,3,3-tetroxid ab, wäscht mit Ether und trocknet, F. 199-200°; MS 507.

Analog erhält man mit 2-Ethoxycarbonyl-1,3-dithian-1,1,3,3-tetroxid das 2-[2-((4S,5S)-3-BOC-4-cyclohexyl-methyl-2,2-dimethyl-5-oxazolidinyl)-ethyl]-2-ethoxy-carbonyl-1,3-dithian-1,1,3,3-tetroxid, FAB 581.

### Beispiel 8

Man kocht ein Gemisch von 4,68 g 2-[(2S,3S)-3-BOC-amino-4-cyclohexyl-2-hydroxy-butyl]-1,3-dithian, 5,83 g 2,2-Dimethoxypropan, 100 ml Toluol und 97 mg p-Toluolsulfonsäure 2 Std., kühlt ab, wäscht mit 2 n NaOH, dann mit Wasser, dampft ein und erhält 2-[(4S,5S)-3-BOC-4-cyclohexylmethyl-2,2-dimethyl-5-oxazolidinylmethyl]-1,3-dithian, F. 104-106°.

### Beispiel 9

Ein Gemisch von 3,89 g 2-[(2S,3S)-3-BOC-amino-4-cyclohexyl-2-hydroxy-butyl]-1,3-dithian, 200 ml Dichlormethan und 15,7 g 55%iger 3-Chlorperbenzoesäure wird 3 Std. bei 20° gerührt. Man filtriert, wäscht das Filtrat mit 2 n NaOH, dann mit gesättigter NaCl-Lösung, arbeitet weiter wie üblich auf und erhält 2-[(2S,3S)-3-BOC-amino-4-cyclohexyl-2-hydroxy-butyl]-1,3-dithian-1,1,3,3-tetroxid, F. 186-188°.

### Beispiel 10

Ein Gemisch von 14,21 g 2-[(4S,5S)-3-BOC-4-cyclohexyl-methyl-2,2-dimethyl-5-oxazolidinyl-methyl]-1,3-dithian, 65 g Monoperoxyphthalsäure-Mg-Salz-hexahydrat und 300 ml THF wird bei 20° 16 Std. gerührt. Man arbeitet mit Ether/verdünnter Natronlauge auf und erhält 2-[(4S,5S)-3-BOC-4-cyclohexylmethyl-2,2-dimethyl-5-oxazolidinyl-methyl]-1,3-dithian-1,1,3,3-tetroxid, F. 219-220°.

### Beispiel 11

Eine Lösung von 1 g 2-[(2S,3S)-3-BOC-amino-4-cyclohexyl-2-hydroxy-butyl]-1,3-dithian in 30 ml mit HCl-Gas gesättigtem Ethylacetat wird 16 Std. bei 0° gerührt. Das ausgefallene 2-[(2S,3S)-3-Amino-4-cyclohexyl-2-hydroxy-butyl]-1,3-dithian-hydrochlorid wird abfiltriert und mit Ethylacetat, dann mit Ether gewaschen. F. 144-147° (Zers.).

Analog erhält man aus den entsprechenden BOC-Verbindungen:
2-[(2S,3S)-3-Amino-4-cyclohexyl-2-hydroxy-butyl]-2-methyl-1,3-dithian
2-[(3S,4S)-4-Amino-5-cyclohexyl-3-hydroxy-pentyl]-1,3-dithian
2-[(3S,4S)-4-Amino-5-cyclohexyl-3-hydroxy-pentyl]-2-methyl-1,3-dithian
sowie die folgenden 1,3-Dithian-1,1,3,3-tetroxide:
2-[(2S,3S)-3-Amino-4-cyclohexyl-2-hydroxy-butyl]-Hydrochlorid, F. 240-242° (Zers.)
2-[(2S,3S)-3-Amino-4-cyclohexyl-2-hydroxy-butyl]-2-methyl-
2-[(2S,3S)-4-Cyclohexyl-2-hydroxy-3-(H-Leu-amino)-butyl]-, Schaum
2-[(2S,3S)-4-Cyclohexyl-2-hydroxy-3-(H-Leu-amino)-butyl]-2-methyl-
2-[(2S,3S)-4-Cyclohexyl-2-hydroxy-3-H-Phe-His-amino-butyl]- , FAB 638
2-[(2S,3S)-4-Cyclohexyl-2-hydroxy-3-H-Phe-His-amino-butyl]-2-methyl-
2-[(2S,3S)-3-(4-Aminopiperidinocarbonyl-Phe-His-amino)-4-cyclohexyl-2-hydroxy-butyl]-, F. 163° (Zers.), FAB 764; Dihydrochlorid, F. 191-195°, FAB 764
2-[(2S,3S)-3-(4-Aminopiperidinocarbonyl-Phe-His-amino)-4-cyclohexyl-2-hydroxy-butyl]-2-methyl-, F. 155° (Zers.), FAB 778
2-[(2S,3S)-3-(4-Aminopiperidinocarbonyl-Phe-Leu-amino)-4-cyclohexyl-2-hydroxy-butyl]-, F. 157° (Zers.), FAB 740; Hydrochlorid, F. 181-184° (Zers.), FAB 740
2-[(2S,3S)-3-(4-Aminopiperidinocarbonyl-Phe-Leu-amino)-4-cyclohexyl-2-hydroxy-butyl]-2-methyl-
2-[(2S,3S)-3-(3-Amino-3-methyl-butyryl)-Mal-His-amino)-4-cyclohexyl-2-hydroxy-butyl]-, FAB 767
2-[(2S,3S)-3-(3-Amino-3-methyl-butyryl)-Mal-His-amino)-4-cyclohexyl-2-hydroxy-butyl]-2-methyl-, FAB 781
2-[(2S,3S)-3-Mal-His-amino-4-cyclohexyl-2-hydroxy-butyl]-2-[(2S,3S)-3-H-Phe-amino-4-cyclohexyl-2-hydroxy-butyl]-
2-[(2S,3S)-3-H-Phe-βAla-amino-4-cyclohexyl-2-hydroxy-butyl]-
2-[(2S,3S)-3-H-Phe-Ile-amino-4-cyclohexyl-2-hydroxy-butyl]-
2-[(2S,3S)-3-H-Phe-Leu-amino-4-cyclohexyl-2-hydroxy-butyl]-
2-[(2S,3S)-3-H-Phe-Nle-amino-4-cyclohexyl-2-hydroxy-butyl]-
2-[(2S,3S)-3-H-Phe-Nva-amino-4-cyclohexyl-2-hydroxy-butyl]-
2-[(2S,3S)-3-H-Phe-Val-amino-4-cyclohexyl-2-hydroxy-butyl]-
2-[(2S,3S)-3-H-Mal―amino-4-cyclohexyl-2-hydroxy-butyl]-
2-[(2S,3S)-3-H-Mal-βAla-amino-4-cyclohexyl-2-hydroxy-butyl]-
2-[(2S,3S)-3-H-Mal-Ile-amino-4-cyclohexyl-2-hydroxy-butyl]-
2-[(2S,3S)-3-H-Mal-Leu-amino-4-cyclohexyl-2-hydroxy-butyl]-
2-[(2S,3S)-3-H-Mal-Nle-amino-4-cyclohexyl-2-hydroxy-butyl]-
2-[(2S,3S)-3-H-Mal-Nva-amino-4-cyclohexyl-2-hydroxy-butyl]-
2-[(2S,3S)-3-H-Mal-Val-amino-4-cyclohexyl-2-hydroxy-butyl]-
2-[(2S,3S)-3-(4-Amino-piperidinocarbonyl-Phe-amino)-4-cyclohexyl-2-hydroxy-butyl]-, F. 147-150°, FAB 627
2-[(2S,3S)-3-(4-Amino-piperidinocarbonyl-Phe-βAla-amino)-4-cyclohexyl-2-hydroxy-butyl]-, F. 167°, FAB 698
2-[(2S,3S)-3-(4-Amino-piperidinocarbonyl-Phe-Ile-amino)-4-cyclohexyl-2-hydroxy-butyl]-
2-[(2S,3S)-3-(4-Amino-piperidinocarbonyl-Phe-Nle-amino)-4-cyclohexyl-2-hydroxy-butyl]-, F. 140-145°, FAB 741,
Hydrochlorid, F. 184-189°, FAB 740
2-[(2S,3S)-3-(4-Amino-piperidinocarbonyl-Phe-Nva-amino)-4-cyclohexyl-2-hydroxy-butyl]-, F. 148-153°, FAB 727,
Hydrochlorid, F. 207-212°, FAB 716
2-[(2S,3S)-3-(4-Amino-piperidinocarbonyl-Phe-Val-amino)-4-cyclohexyl-2-hydroxy-butyl]-, F. 190-192°, FAB 726,
Hydrochlorid, F. 200-205°, FAB 726.
2-[(3S,4S)-4-Amino-5-cyclohexyl-3-hydroxy-pentyl]-
2-[(3S,4S)-4-Amino-5-cyclohexyl-3-hydroxy-pentyl]-2-methyl-
2-[(3S,4S)-5-Cyclohexyl-3-hydroxy-4-H-Phe-His-amino-pentyl]-
2-[(3S,4S)-5-Cyclohexyl-3-hydroxy-4-H-Phe-His-amino-pentyl]-2-methyl-
2-[(3S,4S)-4-(4-Aminopiperidinocarbonyl-Phe-His-amino)-5-cyclohexyl-3-hydroxy-pentyl]-, F. 156-158° (Zers.), FAB 779
2-[(3S,4S)-4-(4-Aminopiperidinocarbonyl-Phe-His-amino)-5-cyclohexyl-3-hydroxy-pentyl]-2-methyl-, F. 185-190°, FAB 792
2-[(3S,4S)-4-(4-Aminopiperidinocarbonyl-Phe-Leu-amino)-5-cyclohexyl-3-hydroxy-pentyl]-
2-[(3S,4S)-4-(4-Aminopiperidinocarbonyl-Phe-Leu-amino)-5-cyclohexyl-3-hydroxy-pentyl]-2-methyl-
2-[(3S,4S)-4-(3-Amino-3-methyl-butyryl-Mal-His-amino)-5-cyclohexyl-3-hydroxy-pentyl]-, F. 188-192°, FAB 781
2-[(3S,4S)-4-(3-Amino-3-methyl-butyryl-Mal-His-amino)-5-cyclohexyl-3-hydroxy-pentyl]-2-methyl-

### Beispiel 12

Eine Lösung von 0,98 g 2-[(4S,5S)-3-BOC-4-cyclohexyl-methyl-2,2-dimethyl-5-oxazolidinyl-methyl]-1,3-dithian-1,1,3,3-tetroxid in 300 ml THF wird bei -30° mit 1,3 ml einer 1,6-molaren Lösung von Butyl-Li in Hexan versetzt. Nach 1std. Rühren gibt man 0,56 g Methyliodid hinzu, rührt 24 Std. im auftauenden Kältebad, verdünnt mit 50 ml Ether, arbeitet wie üblich auf und erhält 2-[(4S,5S)-3-BOC-4-cyclohexylmethyl-2,2-dimethyl-5-oxazolidinyl-methyl]-2-methyl-1,3-dithian-1,1,3,3-tetroxid, F. 176-177°.

### Beispiel 13

Eine Lösung von 1,02 g 2-[2-((4S,5S)-3-BOC-4-cyclohexyl-methyl-2,2-dimethyl-5-oxazolidinyl)-ethyl]-1,3-dithian-1,1,3,3-tetroxid in 30 ml THF wird bei -30° mit 2,6 ml einer 1,6 M C₄H₉Li-Lösung in Hexan versetzt. Man rührt 2 Std. bei -30°, gibt 0,35 ml CH₃I hinzu, rührt 1,5 Std. bei -35°, anschließend 24 Std. bei 20°, arbeitet wie üblich auf und erhält 2-[2-((4S,5S)-3-BOC-4-cyclohexyl-methyl-2,2-dimethyl-5-oxazolidinyl)-ethyl]-2-methyl-1,3-dithian-1,1,3,3-tetroxid, F. 193-196°.

### Beispiel 14

Das nach Beispiel 12 erhaltene Produkt wird mit HCl/Ethylacetat analog Beispiel 12 behandelt und aufgearbeitet. Unter gleichzeitiger Abspaltung der BOC- und der Acetonidgruppe erhält man 2-[(2S,3S)-3-Amino-4-cyclohexyl-2-hydroxy-butyl]-2-methyl-1,3-dithian-1,1,3,3-tetroxid, F. 171-174°.

Analog erhält man aus den entsprechenden N-BOC-oxazolidinen:
2-[(2S,3S)-3-Amino-4-cyclohexyl-2-hydroxy-butyl]-1,3-dithian-1,1,3,3-tetroxid
2-[(3S,4S)-4-Amino-5-cyclohexyl-3-hydroxy-pentyl]-1,3-dithian-1,1,3,3-tetroxid
2-[(3S,4S)-4-Amino-5-cyclohexyl-3-hydroxy-pentyl]-2-methyl-1,3-dithian-1,1,3,3-tetroxid.

### Beispiel 15

Ein Gemisch von 1,19 g Phenylisocyanat, 3,67 g 2-[(2S,3S)-3-Amino-4-cyclohexyl-2-hydroxy-butyl]-2-methyl-1,3-dithian-1,1,3,3-tetroxid, 1,01 g Triethylamin und 60 ml Dichlormethan wird 5 Std. bei 20° gerührt. Nach üblicher Aufarbeitung erhält man 2-[(2S,3S)-4-Cyclohexyl-2-hydroxy-3-(N'-phenylureido)-butyl]-2-methyl-1,3-dithian-1,1,3,3-tetroxid, F. 238-240°, FAB 487.

Analog erhält man aus den entsprechenden Aminoverbindungen die nachstehenden 1,3-Dithian-1,1,3,3-tetroxide:
2-[(2S,3S)-4-Cyclohexyl-2-hydroxy-3-(N'-phenylureido)-butyl]-
2-[(3S,4S)-5-Cyclohexyl-3-hydroxy-4-(N'-phenylureido)-pentyl]-
2-[(3S,4S)-5-Cyclohexyl-3-hydroxy-4-(N'-phenylureido)-pentyl]-2-methyl-
2-[(4S,5S)-4-Cyclohexyl-2,2-dimethyl-3-N-phenylcarbamoyl-5-oxazolidinyl]-
2-[(4S,5S)-4-Cyclohexyl-2,2-dimethyl-3-N-phenylcarbamoyl-5-oxazolidinyl]-2-methyl-, FAB 527
2-[2-((4S,5S)-4-Cyclohexylmethyl-2,2-dimethyl-3-N-phenylcarbamoyl-5-oxazolidinyl)-ethyl]-
2-[2-((4S,5S)-4-Cyclohexylmethyl-2,2-dimethyl-3-N-phenylcarbamoyl-5-oxazolidinyl)-ethyl]-2-methyl-.

### Beispiel 16

Ein Gemisch von 1 g 2-[2-((4S,5S)-3-BOC-4-cyclohexyl-methyl-2,2-dimethyl-5-oxazolidinyl)-ethyl]-2-ethoxy-carbonyl-1,3-dithian-1,1,3,3-tetroxid, 50 ml Dioxan und 20 ml 2 n NaOH (wäßrig) wird 3 Std. bei 20° gerührt. Nach üblicher Aufarbeitung erhält man 2-[2-((4S,5S)-3-BOC-4-cyclohexylmethyl-2,2-dimethyl-5-oxazolidinyl)-ethyl]-2-carboxy-1,3-dithian-1,1,3,3-tetroxid.

### Beispiel 17

Analog Beispiel 2 erhält man aus 2-[(2S,3S)-3-CBZ-amino-4-cyclohexyl-2-hydroxy-butyl]-1,3-dithian-1,1,3,3-tetroxid durch Hydrogenolyse das 2-[(2S,3S)-3-Amino-4-cyclohexyl-2-hydroxy-butyl]-1,3-dithian-1,1,3,3-tetroxid, Hydrochlorid, F. 240-242° (Zers.).

### Beispiel 18

Analog Beispiel 1 erhält man aus den entsprechenden imi-DNP-His-Derivaten die nachstehenden 1,3-Dithian-1,1,3,3-tetroxide:
2-[(2S,3S)-3-(4-BOC-amino-piperidinocarbonyl-Pla-His-amino)-4-cyclohexyl-2-hydroxy-butyl]-, FAB 865
2-[(4S,5S)-5-(BOC-Phe-His-amino)-6-cyclohexyl-4-hydroxy-hexyl]-, F. 150° (Zers.), FAB 766.

### Beispiel 19

Analog Beispiel 3 erhält man aus 2-[(2S,3S)-3-Amino-4-cyclohexyl-2-hydroxy-butyl]-1,3-dithian-1,1,3,3-tetroxid bzw. aus 2-[(3S,4S)-4-Amino-5-cyclohexyl-3-hydroxy-pentyl]-1,3-dithian-1,1,3,3-tetroxid bzw. aus 2-[(4S,5S)-5-Amino-6-cyclohexyl-4-hydroxy-hexyl]-1,3-dithian-1,1,3,3-tetroxid [Dihydrochlorid, F. 209-210°, FAB 382, erhältlich aus (4S,5S)-3-BOC-4-cyclohexyl-methyl-5-(2-methansulfonyloxy-ethyl)-2,2-dimethyl-oxazolidin über die entsprechenden 5-(2-Cyanethyl)-, 5-(2-Carboxyethyl)- (F. 190° (Zers.)), 5-(2-Methoxy carbonyl-ethyl)- (Öl; FAB 384), 5-(3-Hydroxy-propyl)- (F. 60-61°; FAB 356) und 5-(3-Methansulfonyloxy-propyl)-verbindung (F. 63-64°, FAB 434), Reaktion der zuletzt genannten Verbindung mit 1,3-Dithian-1,1,3,3-tetroxid zu 2-[3-((4S,5S)-3-BOC-4-cyclohexylmethyl-2,2-dimethyl-5-oxazolidinyl)-propyl]-1,3-dithian-1,1,3,3-tetroxid (FAB 522) und Spaltung mit methanolischer Salzsäure bei 25°] die folgenden 1,3-Dithian-1,1,3,3-tetroxide:
2-[(3S,4S)-4-(4-BOC-amino piperidinocarbonyl-Phe-βAla-amino)--5-cyclohexyl-3-hydroxy-pentyl]-, F. 143° (Zers.), FAB 812
2-[(3S,4S)-4-BOC-Leu-Met(O₂)-amino-5-cyclohexyl-3-hydroxy-pentyl]-, F. 212-215°, FAB 744
2-[(3S,4S)-4-(4-BOC-amino-piperidinocarbonyl-Phe-amino)-5-cyclohexyl-3-hydroxy-pentyl]-, F. 192° (Zers.), FAB 741
2-[(2S,3S)-3-Anilino-carbonylamino-4-cyclohexyl-2-hydroxy-butyl]-, F. 240-241°, FAB 473
2-[(3S,4S)-4-(4-BOC-amino-piperidinocarbonyl-amino)-5-cyclohexyl-3-hydroxy-pentyl]-, F. 136° (Zers.), FAB 579 (mit 4-BOC-amino-piperidino-carbonylchlorid) 2-[(2S,3S)-3-BOC-Nva-amino-4-cyclohexyl-2-hydroxy-butyl]-, F. 228-230°, FAB 553
2-[(2S,3S)-3-BOC-Nle-amino-4-cyclohexyl-2-hydroxy-butyl]-, F. 225-226°, FAB 567
2-[(2S,3S)-3-BOC-Val-amino-4-cyclohexyl-2-hydroxy-butyl]-, F. 235-236°, FAB 553
2-[(2S,3S)-3-(1-BOC-4-piperidyl-carbonyl-amino)-4-cyclohexyl-2-hydroxy-butyl]-, F. 215-217°, FAB 565
2-[(3S,4S)-4-(4-BOC-amino-pipridinocarbonyl-amino-Phe-Gln-amino)-5-cyclohexyl-3-hydroxy-pentyl]-, F. 170° (Zers.), FAB 869
2-[(2S,3S)-3-(BOC-Met-amino)-4-cyclohexyl-2-hydroxy-butyl]-, F. 233-234°, FAB 585
2-[(2S,3S)-3-(BOC-Met(O₂)-amino)-4-cyclohexyl-2-hydroxy-butyl]-, F. 227-228°, FAB 617
2-[(2S,3S)-3-(4-BOC-amino-piperidinocarbonyl-Phe-Met-amino)-4-cyclohexyl-2-hydroxy-butyl]-, F. 167-169°, FAB 858
2-[(2S,3S)-3-(BOC-(S-Me-Cys)-amino)-4-cyclohexyl-2-hydroxy-butyl]-, F. 222-224°, FAB 571
2-[(3S,4S)-4-(4-BOC-amino-piperidinocarbonyl-Phe-Asn-amino)-5-cyclohexyl-3-hydroxy-pentyl]-, F. 172° (Zers.), FAB 855
2-[(2S,3S)-3-(4-BOC-amino-piperidinocarbonyl-Phe-(S-Me-Cys)-amino)-4-cyclohexyl-2-hydroxy-butyl]-, F. 161-165°, FAB 844
2-[(3S,4S)-4-(BOC-Nva-amino)-5-cyclohexyl-3-hydroxy-pentyl]-, F. 205-208°, FAB 567
2-[(3S,4S)-4-(BOC-Nle-amino)-5-cyclohexyl-3-hydroxy-pentyl]-, F. 208-209°, FAB 581
2-[(2S,3S)-3-(4-BOC-amino-piperidinocarbonyl-Phe-Met(O₂)-amino)-4-cyclohexyl-2-hydroxy-butyl]-, F. 186-188°, FAB 891
2-[(3S,4S)-4-(4-BOC-amino-piperidinocarbonyl-Phe-Nva-amino)-5-cyclohexyl-3-hydroxy-pentyl]-, F. 148° (Zers.). FAB
2-[(2S,3S)-3-(4-BOC-amino-piperidinocarbonyl-Pla-βAla-amino)-4-cyclohexyl-2-hydroxy-butyl]-, FAB 800
2-[(3S,4S)-4-(4-BOC-amino-piperidinocarbonyl-Phe-Nle-amino)-5-cyclohexyl-3-hydroxy-pentyl]-, F. 176° (Zers.), FAB 855
2-[(3S,4S)-4-BOC-Leu-Met-amino-5-cyclohexyl-3-hydroxy-pentyl]-, F. 167-169°, FAB 712
2-[(3S,4S)-4-(4-BOC-amino-piperidinocarbonyl-Pla-(S-Me-Cys)-amino)-5-cyclohexyl-3-hydroxy-pentyl]-, F. 176° (Zers.), FAB 860
2-[(3S,4S)-4-(4-BOC-amino-piperidinocarbonyl-Pla-Nva-amino)-5-cyclohexyl-3-hydroxy-pentyl]-, F. 150° (Zers.), FAB 842
2-[(2S,3S)-3-(2-Benzyl-3-tert.-butyl-sulfonyl-propionyl-Nva-amino)-4-cyclohexyl-2-hydroxy-butyl]-, 2 Isomere, F. 132-138° (Zers.) bzw. F. 121-127°, FAB jeweils 719
2-[(2S,3S)-3-(2-Benzyl-3-tert.-butyl-sulfonyl-propionyl-(S-Me-Cys)-amino)-4-cyclohexyl-2-hydroxy-butyl]-, 2 Isomere. FAB 737 bzw. F. 204-205°, FAB 737
2-[(2S,3S)-3-BOC-Phe-(S-Me-Cys)-amino-4-cyclohexyl-2-hydroxy-butyl)-, F. 215-217°, FAB 718
2-[(2S,3S)-3-(3-BOC-amino-3-methylbutyryl-Phe-(S-Me-Cys)-amino)-4-cyclohexyl-2-hydroxy-butyl]-, F. 211-212°, FAB 818
2-[(2S,3S)-3-(4-BOC-amino-piperidinocarbonyl-Pla-(S-Me-Cys)-amino)-4-cyclohexyl-2-hydroxy-butyl]-, F. 217-219°, FAB 846
2-[(2S,3S)-3-(3-Phenylpropionyl-(S-Me-Cys)-amino)-4-cyclohexyl-2-hydroxy-butyl]-, F. 244-246°, FAB 603
2-[(2S,3S)-3-(4-BOC-amino-piperidinocarbonyl-Pla-Nle-amino)-4-cyclohexyl-2-hydroxy-butyl]-, F. 218-219°, FAB 827
2-[(2S,3S)-3-(3-Phenylpropionyl-Nva-amino)-4-cyclohexyl-2-hydroxy-butyl]-, F. 252-255°, FAB 585
2-[(3S,4S)-4-(3-Phenylpropionyl-(S-Me-Cys)-amino)-5-cyclohexyl-3-hydroxy-pentyl]-, F. 192-198° (Zers.), FAB 617
2-[(3S,4S)-4-(3-Pyridylacetyl-(S-Me-Cys)-amino)-5-cyclohexyl-3-hydroxy-pentyl]-, F. 159-163°, FAB 604
2-[(3S,4S)-4-(4-Pyridylacetyl-(S-Me-Cys)-amino)-5-cyclohexyl-3-hydroxy-pentyl]-, F. 206-207°, FAB 604
2-[(3S,4S)-4-(4-Pyridylmercapto-acetyl-(S-Me-Cys)-amino)-5-cyclohexyl-3-hydroxy-pentyl]-, F. 198-204° (Zers.), FAB 636
2-[(3S,4S)-4-(BOC-Met(O₂)-(S-Me-Cys)-amino)-5-cyclohexyl-3-hydroxy-pentyl]-, F. 202° (Zers.), FAB 748
2-[(3S,4S)-4-(2-Pyrimidinylmercapto-acetyl-(S-Me-Cys)-amino)-5-cyclohexyl-3-hydroxy-pentyl]-, F. 198-200°, FAB 637
2-[(3S,4S)-4-(4-BOC-amino-piperidinocarbonyl-Phe-(S-Me-Cys)-amino)-5-cyclohexyl-3-hydroxy-pentyl]-, F. 151-162°, FAB 858
2-[(2S,3S)-3-(4-BOC-amino-piperidinocarbonyl-Phe-Ser-amino)-4-cyclohexyl-2-hydroxy-butyl]-, F. 155-158°, FAB 814 2-[(2S,3S)-3-(4-Morpholinocarbonyl-Phe-(S-Me-Cys)-amino)-4-cyclohexyl-2-hydroxy-butyl]-, F. 178-180°, FAB 731
2-[(2S,3S)-3-(4-BOC-amino-piperidinocarbonyl-Phe-Isoser-amino)-4-cyclohexyl-2-hydroxy-butyl]-, 2 Isomere, F. 135° bzw. 145°, FAB jeweils 814
2-[(3S,4S)-4-(N-Benzyl-N-methyl-carbamoyl-(S-Me-Cys)-amino)-5-cyclohexyl-3-hydroxy-pentyl]-, F. 180° (Zers.), FAB 646
2-[(3S,4S)-4-(2-Benzyl-3-tert.-butylsulfonyl-propionyl-amino)-5-cyclohexyl-3-hydroxy-pentyl]-, 2 Isomere, FAB jeweils 634
2-[(3S,4S)-4-(2-Pyridyl-acetyl-(S-Me-Cys)-amino)-5-cyclohexyl-3-hydroxy-pentyl]-, F. 152-157° (Zers.), FAB 604
2-[(3S,4S)-4-(3-Anilino-propionyl-(S-Me-Cys)-amino)-5-cyclohexyl-3-hydroxy-pentyl]-, Hydrochlorid, F. 148° (Zers.), FAB 633
2-[(3S,4S)-4-(3-Phenylpropionyl-Nva-amino)-5-cyclohexyl-3-hydroxy-pentyl]-, F. 198-201°, FAB 600
2-[(3S,4S)-4-(2-Pyridylacetyl-Nva-amino)-5-cyclohexyl-3-hydroxy-pentyl]-, F. 130° (Zers.), FAB 586
2-[(3S,4S)-4-(3-Pyridylacetyl-Nva-amino)-5-cyclohexyl-3-hydroxy-pentyl]-, F. 208° (Zers.), FAB 586
2-[(3S,4S)-4-(4-Pyridylacetyl-Nva-amino)-5-cyclohexyl-3-hydroxy-pentyl]-, F. 138° (Zers.), FAB 586
2-[(3S,4S)-4-(4-Pyridylmercaptoacetyl-Nva-amino)-5-cyclohexyl-3-hydroxy-pentyl]-, F. 181° (Zers.), FAB 618
2-[(3S,4S)-4-(N-Benzyl-N-methyl-Gly-Nva-amino)-5-cyclohexyl-3-hydroxy-pentyl]-, F. 198° (Zers.), FAB 628
2-[(3S,4S)-4-(2-Pyrimidinyl-mercapto-acetyl-Nva-amino)-5-cyclohexyl-3-hydroxy-pentyl]-, F. 183° (Zers.), FAB 619
2-[(3S,4S)-4-(3-Anilinopropionyl-Nva-amino)-5-cyclohexyl-3-hydroxy-pentyl]-, F. 170-172°, FAB 614
2-[(4S,5S)-5-(2-Benzyl-3-tert.-butylsulfonyl-propionyl-amino)-6-cyclohexyl-4-hydroxy-hexyl]-, 2 Isomere, F. 218-220° bzw. 182-184°, FAB jeweils 648
2-[(4S,5S)-5-(BOC-Phe-(S-Me-Cys)-amino)-6-cyclohexyl-4-hydroxy-hexyl]-, F. 193-195°, FAB 746
2-[(2S,3S)-3-(2-Benzyl-3-tert.-butylsulfonyl-propionyl-amino)-4-cyclohexyl-2-hydroxy-butyl]-, 2 Isomere, F. 170-171° bzw. 230-233°, FAB jeweils 620
2-[(4S,5S)-5-(2-Benzyl-3-tert.-butylsulfonyl-propionyl-Nle-amino)-6-cyclohexyl-4-hydroxy-hexyl]-, 2 Isomere, F. 122-124° (Zers.) bzw. 218-221° (Zers.), FAB jeweils 761
2-[(4S,5S)-5-(3-BOC-amino-3-methyl-butyryl-amino)-6-cyclohexyl-4-hydroxy-hexyl]-, F. 91° (Zers), FAB 581
2-[(4S,5S)-5-(3-BOC-amino-3-methyl-butyryl-Nva-amino)-6-cyclohexyl-4-hydroxy-hexyl]-, F. 168-171° (Zers.), FAB 680
2-[(4S,5S)-5-(2-Benzyl-3-tert.-butylsulfonyl-propionyl-(S-Me-Cys)-amino)-6-cyclohexyl-4-hydroxy-hexyl]-, 2 Isomere, F. 222-223° bzw. 111-113°, FAB jeweils 765
2-[(4S,5S)-5-(4-BOC-amino-piperidinocarbonyl-Phe-βAla-amino)-6-cyclohexyl-4-hydroxy-hexyl]-, F. 128-130°, FAB 826
2-[(4S,5S)-5-(2-Benzyl-3-tert.-butylsulfonyl-propionyl-Nva-amino)-6-cyclohexyl-4-hydroxy-hexyl]-, FAB 761
2-[(2S,3S)-3-(BOC-(S-Me-Cys)-(S-Me-Cys)-amino)-4-cyclohexyl-2-hydroxy-butyl]-, F. 201-202°, FAB 688
2-[(4S,5S)-5-(4-BOC-amino-piperidinocarbonyl-Phe-Nva-amino)-6-cyclohexyl-4-hydroxy-hexyl]-, F. 134-139°, FAB 854
2-[(4S,5S)-5-(4-BOC-amino-piperidinocarbonyl-Phe-Nle-amino)-6-cyclohexyl-4-hydroxy-hexyl]-, F. 135-140°, FAB 868
2-[(2S,3S)-3-(4-Phenyl-4-piperidinylcarbonyl-(S-Me-Cys)-amino)-4-cyclohexyl-2-hydroxy-butyl]-, F. 163-166° (Zers.), FAB 658
2-[(3S,4S)-4-(BOC-Ser-(S-Me-Cys)-amino)-5-cyclohexyl-3-hydroxy-pentyl]-, F. 114° (Zers.)
2-[(3S,4S)-4-(4-Phenyl-2-pyridyl-carbonyl-(S-Me-Cys)-amino)-5-cyclohexyl-3-hydroxy-pentyl]-, Hydrochlorid, F. 153° (Zers.), FAB 666
2-[(3S,4S)-4-(4-Phenyl-3-pyridyl-carbonyl-(S-Me-Cys)-amino)-5-cyclohexyl-3-hydroxy-pentyl]-, Hydrochlorid, F. 158° (Zers.), FAB 666
2-[(3S,4S)-4-(5-p-Fluorphenyl-3-pyridyl-carbonyl-(S-Me-Cys)-amino)-5-cyclohexyl-3-hydroxy-pentyl]-, Hydrochlorid, FAB 685 2-[(2S,3S)-3-(Phenylacetyl-(S-Me-Cys)-amino)-4-cyclohexyl-2-hydroxy-butyl]-, F. 237-239°, FAB 589
2-[(3S,4S)-4-(4-BOC-amino-piperidinocarbonyl-Phe-Thr-amino)-5-cyclohexyl-3-hydroxy-pentyl]-, F. 138-146° (Zers.), FAB 842.

### Beispiel 20

Analog Beispiel 11 erhält man aus den entsprechenden BOC-Verbindungen die folgenden 1,3-Dithian-1,1,3,3-tetroxide:
2-[(3S,4S)-4-(4-Amino-piperidinocarbonyl-Phe-βAla-amino)-5-cyclohexyl-3-hydroxy-pentyl]-, F. 187° (Zers.), FAB 712
2-[(3S,4S)-4-(4-Amino-piperidinocarbonyl-Phe-amino)-5-cyclohexyl-3-hydroxy-pentyl]-, F. 164° (Zers.), FAB 641
2-[(3S,4S)-4-(4-Piperidylcarbonyl-amino)-5-cyclohexyl-3-hydroxy-pentyl]-, F. 167° (Zers.), FAB 479
2-[(2S,3S)-3-(4-Piperidylcarbonyl-amino)-4-cyclohexyl-3-hydroxy-butyl]-, F. 244° (Zers.), FAB 465
2-[(3S,4S)-4-(4-Amino-piperidinocarbonyl-Phe-Gln-amino)-5-cyclohexyl-3-hydroxy-pentyl]-, F. 193° (Zers.), FAB 769
2-[(3S,4S)-4-(4-Amino-piperidinocarbonyl-Phe-Asn-amino)-5-cyclohexyl-3-hydroxy-pentyl]-, F. 198° (Zers.), FAB 755
2-[(3S,4S)-4-(H-Nle-amino)-5-cyclohexyl-3-hydroxy-pentyl]-, F. 180° (Zers.), FAB 481
2-[(2S,3S)-3-(4-Amino-piperidinocarbonyl-Phe-(S-Me-Cys)-amino)-4-cyclohexyl-2-hydroxy-butyl]-, F. 142-145°, FAB 745
2-[(2S,3S)-3-(4-Amino-piperidinocarbonyl-Pla-His-amino)-4-cyclohexyl-2-hydroxy-butyl]-, F. 130-133°, FAB 766
2-[(3S,4S)-4-(4-Amino-piperidinocarbonyl-Phe-Nva-amino)-5-cyclohexyl-3-hydroxy-pentyl]-, F. 192° (Zers.), FAB 740
2-[(2S,3S)-3-(4-Amino-piperidinocarbonyl-Phe-Met-amino)-4-cyclohexyl-2-hydroxy-butyl]-, F. 175° (Zers.), FAB 758
2-[(2S,3S)-3-(4-Amino-piperidinocarbonyl-Phe-Met(O₂)-amino)-4-cyclohezyl-2-hydroxy-butyl]-, F. 198° (Zers.), FAB 790
2-[(2S,3S)-3-(4-Amino-piperidinocarbonyl-Pla-βAla-amino)-4-cyclohexyl-2-hydroxy-butyl]-, FAB 699
2-[(3S,4S)-4-(4-Amino-piperidinocarbonyl-Phe-Nle-amino)-5-cyclohexyl-3-hydroxy-pentyl]-, F. 178° (Zers.), FAB 754
2-[(3S,4S)-4-(4-Amino-piperidinocarbonyl-Pla-(S-Me-Cys)-amino)-5-cyclohexyl-3-hydroxy-pentyl]-, FAB 759
2-[(2S,3S)-3-H-Phe-(S-Me-Cys)-amino-4-cyclohexyl-2-hydroxy-butyl]-, F. 208-210°, FAB 618
2-[(3S,4S)-4-(4-Amino-piperidinocarbonyl-Pla-Nva-amino)-5-cyclohexyl-3-hydroxy-pentyl]-, F. 176° (Zers.), FAB 741
2-[(3S,4S)-4-(H-Met(O₂)-(S-Me-Cys)-amino)-5-cyclohexyl-3-hydroxy-pentyl]-, F. 153° (Zers.), FAB 648
2-[(3S,4S)-4-(4-Amino-piperidinocarbonyl-Phe-(S-Me-Cys)-amino)-5-cyclohexyl-3-hydroxy-pentyl]-, F. 172° (Zers.), FAB 758
2-[(2S,3S)-3-(3-Amino-3-methyl-butyryl-Phe-(S-Me-Cys)-amino)-4-cyclohexyl-2-hydroxy-butyl]-, F. 172-175°, FAB 717
2-[(2S,3S)-3-(4-Amino-piperidinocarbonyl-Phe-Met-amino)-4-cyclohexyl-2-hydroxy-butyl]-, Hydrochlorid, F. 185° (Zers.), FAB 758
2-[(2S,3S)-3-(4-Amino-piperidinocarbonyl-Phe-Ser-amino)-4-cyclohexyl-2-hydroxy-butyl]-, Hydrochlorid, F. 194° (Zers.), FAB 714
2-[(2S,3S)-3-(4-Amino-piperidinocarbonyl-Pla-(S-Me-Cys)-amino-4-cyclohexyl-2-hydroxy-butyl]-, Hydrochlorid, F. 220° (Zers.), FAB 745
2-[(2S,3S)-3-(4-Amino-piperidinocarbonyl-Pla-Nva-amino)-4-cyclohexyl-2-hydroxy-butyl]-, Hydrochlorid, F. 215° (Zers.), FAB 727
2-[(4S,5S)-5-(4-Amino-piperidinocarbonyl-Phe-Nle-amino)-6-cyclohexyl-4-hydroxy-hexyl]-, Trifluoracetat, F. 138-139°, FAB 768
2-[(4S,5S)-5-(4-Amino-piperidinocarbonyl-Phe-Nva-amino)-6-cyclohexyl-4-hydroxy-hexyl]-, Trifluoracetat, F. 133-135°, FAB 754
2-[(2S,3S)-3-(4-Amino-piperidinocarbonyl-Pla-His-amino)-4-cyclohexyl-2-hydroxy-butyl]-, Dihydrochlorid, F. 216°, FAB 765.

### Beispiel 21

Analog Beispiel 6 erhält man aus (4S,5S)-3-BOC-4-cyclohexyl-methyl-2,2-dimethyl-5-oxazolidinyl-carbaldehyd und 1,3-Propandithiol das 2-[(1S,2S)-2-BOC-amino-3-cyclohexyl-1-hydroxy-propyl]-1,3-dithian, FAB 376.

### Beispiel 22

Ein Gemisch von 1 g 2-[(1S,2S)-2-BOC-amino-3-cyclohexyl-1-hydroxy-propyl]-1,3-dithian, 5 ml 2,2-Dimethoxypropan, 0.1 g p-Toluolsulfonsäure und 50 ml Dichlormethan wird 16 Std. bei 20° gerührt. Man extrahiert mit NaHCO₃-Lösung, wäscht neutral, dampft ein und erhält 2-[(4S,5S)-3-BOC-4-cyclohexylmethyl-2,2-dimethyl-5-oxazolidinyl]-1,3-dithian.

### Beispiel 23

Eine Lösung von 0,51 g 2-[2-((4S,5S)-3-BOC-4-cyclohexyl-methyl-2,2-dimethyl-5-oxazolidinyl)-ethyl]-1,3-dithian-1,1,3,3-tetroxid in 15 ml THF wird unter N₂ bei -40° mit 1.7 ml einer 1,6 molaren Lösung von Butyllithium in Hexan versetzt. Nach 1std. Rühren gibt man 0.26 ml 3-Brompropionsäureethylester hinzu, rührt 1 Std. bei -40° und 24 Std. bei 25° und arbeitet wie üblich (Wasser/Ether) auf. Man erhält 2-[2-((4S,5S)-3-BOC-4-cyclohexylmethyl)-2,2-dimethyl-5-oxazolidinyl)-ethyl] -2-(2-ethoxycarbonylethyl)-1,3-dithian-1,1,3,3-tetroxid, F. 160-161°, FAB 608.

Die nachstehenden Beispiele betreffen pharmazeutische Zubereitungen.

### Beispiel A: Tabletten

Ein Gemisch von 1 kg 2-[(2S,3S)-3-BOC-Phe-His-amino-4-cyclohexyl-2-hydroxy-butyl]-1,3-dithian-1,1,3,3-tetroxid, 4 kg Lactose, 1,2 kg Maisstärke, 200 g Talk und 100 g Magnesiumstearat wird in üblicher Weise zu Tabletten verpreßt, derart daß jede Tablette 100 mg Wirkstoff enthält.

### Beispiel B: Dragees

Analog Beispiel A werden Tabletten gepreßt, die anschließend in üblicher Weise mit einem Überzug aus Saccharose, Maisstärke, Talk, Tragant und Farbstoff überzogen werden.

### Beispiel C: Kapseln

500 g 2-[(2S,3S)-3-(4-Aminopiperidinocarbonyl-Phe-His-amino)-4-cyclohexyl-2-hydroxy-butyl]-1,3-dithian-1,1,3,3-tetroxid werden in üblicher Weise in Hartgelatinekapseln gefüllt, so daß jede Kapsel 500 mg Wirkstoff enthält.

### Beispiel D: Injektionsgläser

Eine Lösung von 100 g 2-[(2S,3S)-3-(4-Aminopiperidinocarbonyl-Phe-His-amino)-4-cyclohexyl-2-hydroxy-butyl]-1,3-dithian-1,1,3,3-tetroxid in 4 l zweifach destilliertem Wasser wird mit 2n Salzsäure auf pH 6,5 eingestellt, steril filtriert und in Injektionsgläser abgefüllt. Man lyophilisiert unter sterilen Bedingungen und verschließt steril. Jedes Injektionsglas enthält 50 mg Wirkstoff.

### Beispiel E: Suppositorien

Man schmilzt ein Gemisch von 50 g 2-[(2S,3S)-3-(4-BOC-amino-piperidinocarbonyl-Phe-amino)-4-cyclohexyl-2-hydroxy-butyl]-1,3-dithian-1,1,3,3-tetroxid mit 10 g Sojalecithin und 140 g Kakaobutter, gießt in Formen und läßt erkalten. Jedes Suppositorium enthält 250 mg Wirkstoff.

## Patentansprüche

1. Peptid-Analoga der Formel I
R¹-Z-NR²-CHR³-CR⁴-(CH₂)ₒ-(CR⁵)ₜ-CE-C_{w}H_{2w}-R⁶ I
worin
R¹ H, R⁷-CₘH₂ₘ-O-CO-, R⁷-CₘH₂ₘ-CO-, R⁸R⁹N-CₘH₂ₘ-CO- oder R¹¹-CₘH₂ₘ-(T)ₛ-(V)_{y}-CₙH₂ₙ-L(R⁷-CₚH₂ₚ)-CᵣH₂ᵣ-CO-,
Z 0 bis 2 peptidartig miteinander verbundene Aminosäurereste ausgewählt aus der Gruppe bestehend aus βAla, Asn, Asp, Cal, S-A-Cys, Gln, His, Ile, Isoser, Leu, Mal, Met, Met(O₂), Nle, Nva, Phe, Ser, Thr und Val, wobei an Stelle eines dieser Reste auch Pla stehen kann,
R² H,
R³ Cyclohexylmethyl,
R⁴ (H, R¹²),
R⁵ (H, H) oder (H, OH),
R⁶ H, A oder COOR⁷,
R⁷ und R¹¹ jeweils H, A, Ar oder Het,
R⁸R⁹N eine unsubstituierte oder eine durch NH₂ oder NH-CO-O-A substituierte Piperidino- oder Morpholinogruppe,
R¹¹ auch R⁸OOC-,
R¹² OH,
R² und R¹² zusammen auch -C(A)₂-O-,
E -S(O)_{b}-(CH₂)₃-S(O)_{b}-,
L CH oder N,
T NH,
V S oder SO₂,
a 2 oder 3,
b 0 oder 2,
s, t und y jeweils 0 oder 1,
m, n, o, p und w jeweils 0, 1, 2, 3 oder 4,
Ar unsubstituiertes oder einfach durch Hal substituiertes Phenyl,
Het einen gesättigten oder ungesättigten 5- oder 6-gliedrigen heterocyclischen Rest mit 1-4 N-Atomen, der einfach durch Hal oder Ar substituiert sein kann,
Hal F, Cl, Br oder J und
A Alkyl mit 1-8 C-Atomen bedeuten,
sowie deren Salze.

2. a) 2-[(2S,3S)-3-(BOC-Phe-His-amino)-4-cyclohexyl-2-hydroxy-butyl]-1,3-dithian;
b) 2-[(2S,3S)-3-(BOC-Phe-His-amino)-4-cyclohexyl-2-hydroxy-butyl]-1,3-dithian-1,1,3,3-tetroxid.

3. Verfahren zur Herstellung einer Verbindung der Formel I gemäß Patentanspruch 1 sowie von ihren Salzen, dadurch gekennzeichnet, daß man sie aus einem ihrer funktionellen Derivate durch Behandeln mit einem solvolysierenden oder hydrogenolysierenden Mittel in Freiheit setzt oder daß man eine Carbonsäure der Formel II
R¹-G¹-OH II
worin
G¹
(a) fehlt,
(b) Z,
(c) Z¹ bedeutet
oder eines ihrer reaktionsfähigen Derivate mit einer Aminoverbindung der Formel III
H-G²-NR²-CHR³-CR⁴-(CH₂)ₒ-(CR⁵)ₜ-CE-C_{w}H_{2w}-R⁶ III
worin
G²
(a) Z bedeutet,
(b) fehlt,
(c) Z² bedeutet und
Z¹ und Z² zusammen Z bedeuten,
umsetzt,
oder daß man zur Herstellung einer Verbindung der Formel I, worin b = 0 ist, eine Carbonylverbindung der Formel IV
R¹-Z-NR²-CHR³-CR⁴-(CH₂)ₒ-(CR⁵)ₜ-CO-C_{w}H_{2w}-R⁶ IV
mit einem Dithiol der Formel V
HS-(CH₂)₃-SH V
oder mit einem seiner Salze
umsetzt,
oder daß man zur Herstellung einer Verbindung der Formel I, worin E = -SO₂-(CH₂)₃-SO₂- bedeutet, eine Verbindung der Formel VI
R¹-Z-NR²-CHR³-CR⁴-(CH₂)ₒ-(CR⁵)ₜ-X VI
worin
X Cl, Br, I oder eine reaktionsfähig veresterte OH-Gruppe bedeutet,
mit einer Verbindung der Formel VII
H-CE-C_{w}H_{2w}-R⁶ VII
umsetzt
und daß man gegebenenfalls eine Verbindung der Formel I in eine andere Verbindung der Formel I umwandelt und/oder eine Verbindung der Formel I durch Behandeln mit einer Säure oder Base in eines ihrer Salze überführt.

4. Verwendung einer Verbindung der Formel I gemäß Patentanspruch 1 oder eines ihrer physiologisch unbedenklichen Salze zur Herstellung pharmazeutischer Präparate.

5. Pharmazeutisches Präparat, enthaltend eine Verbindung der Formel I gemäß Patentanspruch 1 oder eines ihrer physiologisch unbedenklichen Salze.

## Claims

1. Peptide analogues of the formula I
R¹-Z-NR²-CHR³-CR⁴-(CH₂)ₒ-(CR⁵)ₜ-CE-C_{w}H_{2w}-R⁶ I
in which
R¹ is H, R⁷-CₘH₂ₘ-O-CO-, R⁷-CₘH₂ₘ-CO-, R⁸R⁹N-CₘH₂ₘ-CO- or R¹¹-CₘH₂ₘ-(T)ₛ-(V)_{y}-CₙH₂ₙ-L(R⁷-CₚH₂ₚ)-CᵣH₂ᵣ-CO-,
Z is 0 to 2 amino acid residues which are linked together in the manner of a peptide and are selected from the group comprising βAla, Asn, Asp, Cal, S-A-Cys, Gln, His, Ile, Isoser, Leu, Mal, Met, Met(O₂), Nle, Nva, Phe, Ser, Thr and Val, Pla also being able to stand in place of one of these residues,
R² is H,
R³ is cyclohexylmethyl,
R⁴ is (H, R¹²),
R⁵ is (H, H) or (H, OH),
R⁶ is H, A or COOR⁷,
R⁷ and R¹¹ are in each case H, A, Ar or Het,
R⁸R⁹N is a piperidino or morpholino group which is unsubstituted or substituted by NH₂ or NH-CO-O-A,
R¹¹ is also R⁸OOC-,
R¹² is OH,
R² and R¹² together are also -C(A)₂-O-,
E is -S(O)_{b}-(CH₂)₃-S(O)_{b}-,
L is CH or N,
T is NH,
V is S or SO₂,
a is 2 or 3,
b is 0 or 2,
s, t and y are each 0 or 1,
m, n, o, p and w are each 0, 1, 2, 3 or 4,
Ar is phenyl which is unsubstituted or is substituted once by Hal,
Het is a saturated or unsaturated 5- or 6-membered heterocyclic radical which has 1-4 N atoms and can be substituted once by Hal or Ar,
Hal is F, Cl, Br or I, and
A is alkyl having 1-8 C atoms,
and the salts thereof.

2. a) 2-[(2S,3S)-3-(BOC-Phe-His-amino)-4-cyclohexyl-2-hydroxy-butyl]-1,3-dithiane;
b) 2-[(2S,3S)-3-(BOC-Phe-His-amino)-4-cyclohexyl-2-hydroxy-butyl]-1,3-dithiane 1,1,3,3-tetroxide.

3. Process for the preparation of a compound of the formula I according to Patent Claim 1, and of the salts thereof, characterized in that it is liberated from one of its functional derivatives by treatment with a solvolyzing or hydrogenolyzing agent, or in that a carboxylic acid of the formula II
R¹-G¹-OH II
in which G¹ is
(a) absent,
(b) Z,
(c) Z¹,
or one of the reactive derivatives thereof, is reacted with an amino compound of the formula III
H-G²-NR²-CHR³-CR⁴-(CH₂)ₒ-(CR⁵)ₜ-CE-C_{w}H_{2w}-R⁶ III
in which G² is
(a) Z,
(b) absent,
(c) Z² and
Z¹ + Z² are together Z,
or in that, for preparing a compound of the formula I in which b is 0, a carbonyl compound of the formula IV
R¹-Z-NR²-CHR³-CR⁴-(CH₂)ₒ-(CR⁵)ₜ-CO-C_{w}H_{2w}-R⁶ IV
is reacted with a dithiol of the formula V
HS-(CH₂)₃-SH V
or with one of its salts, or in that, for preparing a compound of the formula I in which E is -SO₂-(CH₂)₃-SO₂-, a compound of the formula VI
R¹-Z-NR²-CHR³-CR⁴-(CH₂)ₒ-(CR⁵)ₜ-X VI
in which
X is Cl, Br, I or an OH group which is esterified in a reactive way,
is reacted with a compound of the formula VII
H-CE-C_{w}H_{2w}-R⁶ VII
and in that, where appropriate, a compound of the formula I is converted into another compound of the formula I, and/or a compound of the formula I is converted by treatment with an acid or base into one of the salts thereof.

4. Use of a compound of the formula I according to Patent Claim 1 or of one of its physiologically acceptable salts for the preparation of pharmaceutical products.

5. Pharmaceutical product containing a compound of the formula I according to Patent Claim 1 or one of its physiologically acceptable salts.

## Revendications

1. Analogues peptidiques de formule I
R¹-Z-NR²-CHR³-CR⁴-(CH₂)ₒ-(CR⁵)ₜ-CE-C_{w}H_{2w}-R⁶ I
où
R¹ représente H, R⁷-CₘH₂ₘ-O-CO-, R⁷-CₘH₂ₘ-CO-, R⁸R⁹N-CₘH₂ₘ-CO ou R¹¹-CₘH₂ₘ-(T)ₛ-(V)_{y}-CₙH₂ₙ-L R⁷-CₚH₂ₚ)-CᵣH₂ᵣ-CO-,
Z 0 à 2 restes d'acides aminés reliés à la façon des peptides, choisis dans le groupe constitué par βAla, Asn, Asp, Cal, S-A-Cys, Gln, His, Ile, Isoser, Leu, Mal, Met, Met(O₂), Nle, Nva, Phe, Ser, Thr et Val, Pla pouvant remplacer l'un de ces restes,
R² H,
R³ le cyclohexylméthyle,
R⁴ (H, R¹²),
R⁵ (H,H) ou (H,OH),
R⁶ H, A ou COOR⁷,
R⁷ et R¹¹ représentent H, A, Ar ou Het,
R⁸R⁹N représente un groupe pipéridino ou morpholino non non substitué ou substitué par NH₂ ou NH-CO-O-A,
R¹¹ représente également R⁸OOC-,
R¹² OH,
R² et R¹² représentent ensemble également -C(A)₂-O-,
E -S(O)_{b}-(CH₂)₃-S(O)_{b}-,
L CH ou N,
T NH,
V S ou SO₂,
a 2 ou 3,
b 0 ou 2,
s, t et y 0 ou 1,
m, n, o, p et W 0, 1, 2, 3 ou 4,
Ar un phényle non substitué ou monosubstitué par Hal,
Het un reste hétérocyclique à 5 ou 6 chaînons saturé ou insaturé comportant 1 à 4 atomes de N pouvant être monosubstitué par Hal ou Ar,
Hal F, Cl, Br ou I et
A un alkyle comportant 1 à 8 atomes de C,
ainsi que leurs sels.

2. a) le 2-[(2S,3S)-3-(BOC-Phe-His-amino)-4-cyclohexyl-2-hydroxy-butyl]-1,3-dithiane,
b) le 2-[(2S,3S)-3-(BOC-His-amino)-4-cyclohexyl-2-hydroxy-butyl]-1,3-dithiane-1,1,3,3-tétroxyde.

3. Procédé pour la préparation d'un composé de formule I selon la revendication 1, ainsi que de ses sels, caractérisé en ce que l'on libère ces composés d'un de leurs dérivés fonctionnels par traitement avec un agent solvolysant ou hydrogénolysant ou en ce que l'on fait réagir un acide carboxylique de formule II
R¹-G¹-OH II
où G¹
(a) manque
(b) représente Z,
(c) représente Z¹
ou l'un de ses dérivés réactifs avec un composé aminé de formule III
H-G²-NR²-CHR³-CR⁴-(CH₂)ₒ-(CR⁵)ₜ-CE-C_{w}H_{2w}-R⁶ III
où G²
(a) représente Z
(b) manque,
(c) représente Z² et
Z¹ et Z² représentent ensemble Z, ou en ce que, pour la préparation d'un composé de formule I où b = 0, on fait réagir un composé carbonylé de formule IV
R¹-Z-NR²-CHR³-CR⁴-(CH₂)ₒ-(CR⁵)ₜ-CO-C_{w}H_{2w}-R⁶ IV
avec un dithiol de formule V
HS-(CH₂)₃-SH V
ou avec l'un de ses sels,
ou en ce que, pour la préparation d'un composé de formule I où E = -SO₂-(CH₂)₃-SO₂-, on fait réagir un composé de formule VI
R¹-Z-NR²-CHR³-CR⁴-(CH₂)ₒ-(CR⁵)ₜ-X VI
où
X représente Cl, Br, I ou un groupe OH estérifié réactif,
avec un composé de formule VII
H-CE-C_{w}H_{2w}-R⁶ VII
et en ce que l'on transforme éventuellement un composé de formule I en un autre composé de formule I et/ou en ce que l'on transforme un composé de formule I par traitement avec un acide ou une base en l'un de ses sels.

4. Utilisation d'un composé de formule I selon la revendication 1 ou de l'un de ses sels physiologiquement acceptables pour la préparation de compositions pharmaceutiques.

5. Préparation pharmaceutique contenant un composé de formule I selon la revendication 1 ou l'un de ses sels physiologiquement acceptables.
